(19) **Europäisches Patentamt**
**European Patent Office**
**Office européen des brevets**

(11) **EP 4 654 205 A1**

(12) **EUROPEAN PATENT APPLICATION**

(43) Date of publication:
   **26.11.2025 Bulletin 2025/48**

(21) Application number: **25178122.5**

(22) Date of filing: **22.05.2025**

(51) International Patent Classification (IPC):
   *G16B 15/20* (2019.01)   *G06N 3/045* (2023.01)
   *G16B 15/30* (2019.01)   *G16B 20/00* (2019.01)
   *G16B 40/20* (2019.01)   *G06N 3/084* (2023.01)

(52) Cooperative Patent Classification (CPC):
   **G16B 15/30; G06N 3/04; G06N 3/044; G06N 3/045;
   G06N 3/0455; G06N 3/0464; G06N 3/047;
   G06N 3/048; G06N 3/08; G06N 3/084; G06N 3/088;
   G06N 3/09; G06N 5/01; G16B 15/20; G16B 20/00;**
   (Cont.)

(84) Designated Contracting States:
   **AL AT BE BG CH CY CZ DE DK EE ES FI FR GB
   GR HR HU IE IS IT LI LT LU LV MC ME MK MT NL
   NO PL PT RO RS SE SI SK SM TR**
   Designated Extension States:
   **BA**
   Designated Validation States:
   **GE KH LA MA MD TN**

(30) Priority: **22.05.2024 US 202463650712 P**

(71) Applicant: **Isomorphic Labs Limited
   London EC2M 4RB (GB)**

(72) Inventors:
   • **Jaderberg, Maxwell Elliot**
     **London, EC2M 4RB (GB)**
   • **Czarnecki, Wojciech**
     **London, EC2M 4RB (GB)**
   • **Ashukha, Arsenii**
     **London, EC2M 4RB (GB)**
   • **Rózemberczki, Benedek Andras**
     **London, EC2M 4RB (GB)**
   • **Ayoola, Thomas**
     **London, EC2M 4RB (GB)**
   • **Briody, Joss William**
     **London, EC2M 4RB (GB)**
   • **Malinin, Andrey**
     **London, EC2M 4RB (GB)**
   • **Kirkpatrick, James**
     **London, EC2M 4RB (GB)**

(74) Representative: **Marks & Clerk GST
   1 New York Street
   Manchester M1 4HD (GB)**

Remarks:
   CORRECT PARTS INCLUDED UNDER RULE
   56a(4) EPC

(54) **PREDICTING PROPERTIES OF PROTEINS AND LIGANDS**

(57)   Methods, systems, and apparatus, including computer programs encoded on a computer storage medium, for generating a predicted property score of a protein and a ligand. **In** one aspect, a method comprises: obtaining a network input that characterizes a protein and a ligand; processing the network input characterizing the protein and the ligand using an embedding neural network to generate a protein-ligand embedding representing the protein and the ligand, wherein the embedding neural network has been jointly trained with a generative model that is configured to: receive an input protein-ligand embedding; and generate, while conditioned on the input protein-ligand embedding, a predicted joint three-dimensional (3D) structure of an input protein and an input ligand represented by the input protein-ligand embedding; and generating a property score that defines a predicted property of the protein and the ligand using the protein-ligand embedding.

FIG. 1A

# EMBEDDING NEURAL NETWORK 112

PROTEIN DATA
102

LIGAND DATA 104

PROTEIN EMBEDDING
NEURAL NETWORK 120

LIGAND EMBEDDING
NEURAL NETWORK 122

PROTEIN
EMBEDDING 124

LIGAND
EMBEDDING 126

FUSION NEURAL NETWORK 128

PROTEIN – LIGAND
EMBEDDING 106

FIG. 1B

# GENERATING JOINT EMBEDDING OF PROTEIN AND LIGAND

130

RECEIVE PROTEIN EMBEDDING AND LIGAND EMBEDDING — 132

CONCATENATE PROTEIN EMBEDDING AND LIGAND EMBEDDING INTO 1D SEQUENCE OF EMBEDDINGS — 134

TRANSFORM 1D SEQUENCE OF EMBEDDINGS INTO 2D ARRAY OF EMBEDDINGS — 136

PROCESS 2D ARRAY OF EMBEDDINGS USING SELF-ATTENTION LAYERS OF FUSION NEURAL NETWORK — 138

OUTPUT 2D ARRAY OF EMBEDDINGS GENERATED BY FUSION NEURAL NETWORK AS PROTEIN – LIGAND EMBEDDING — 140

FIG. 1C

FIG. 1D

(52) Cooperative Patent Classification (CPC): (Cont.)
G16B 40/20

**EP 4 654 205 A1**

**Description**

BACKGROUND

This specification relates to predicting one or more properties of a ligand and a protein.

Predictions can be made using machine learning models. Machine learning models receive an input and generate an output, e.g., a predicted output, based on the received input. Some machine learning models are parametric models and generate the output based on the received input and on values of the parameters of the model. Some machine learning models are deep models that employ multiple layers of models to generate an output for a received input. For example, a deep neural network is a deep machine learning model that includes an output layer and one or more hidden layers that each apply a non-linear transformation to a received input to generate an output.

SUMMARY

This specification describes a system implemented as computer programs on one or more computers in one or more locations that can predict a property of a ligand and a protein.

A "protein" can be understood to refer to any biological molecule that is specified by one or more sequences (or "chains") of amino acids. For example, the term protein can refer to a protein domain, e.g., a portion of an amino acid chain of a protein that can undergo protein folding nearly independently of the rest of the protein. As another example, the term protein can refer to a protein complex, i.e., that includes multiple amino acid chains that jointly fold into a protein structure.

A "ligand" can refer to a molecule or compound that binds to a target molecule, e.g., a protein. Ligands can include, e.g., small organic molecules, complex organic molecules, proteins, biomolecules (e.g., polynucleotides or polypeptides), and so forth.

A "multiple sequence alignment" (MSA) for an amino acid chain in a protein specifies a sequence alignment of the amino acid chain with multiple additional amino acid chains, e.g., from other proteins, e.g., homologous proteins. More specifically, the MSA can define a correspondence between the positions in the amino acid chain and corresponding positions in multiple additional amino acid chains. A MSA for an amino acid chain can be generated, e.g., by processing a database of amino acid chains using any appropriate computational sequence alignment technique, e.g., progressive alignment construction. The amino acid chains in the MSA can be understood as having an evolutionary relationship, e.g., where each amino acid chain in the MSA may share a common ancestor. The correlations between the amino acids in the amino acid chains in a MSA for an amino acid chain can encode information that is relevant to predicting the structure of the amino acid chain.

A "binding pocket" on a protein can refer to a specific three-dimensional cavity or crevice within the structure of the protein where a ligand can bind to the protein. The binding pocket can, in some cases, be understood as a "lock" that fits the shape and chemical properties of ligands that act as "keys" for the lock. In other cases, the ligand may initially not fit perfectly into the binding pocket, e.g., due to structural differences or slight mismatches in shape or chemical groups, but conformational changes during binding can cause the interaction between the ligand and the binding pocket to become more complementary and specific, e.g., as in induced-fit binding. Examples of binding pockets include, e.g., orthosteric binding pockets, allosteric binding pockets, and cryptic binding pockets.

A first neural network can be referred to as a "subnetwork" of a second neural network if the first neural network is included in the second neural network.

A "block" (e.g., a "self-attention block") in a neural network can refer to a group of one or more neural network layers in the neural network.

An "embedding" of an entity (e.g., an atom, or a ligand, or a protein) can refer to a representation of the entity as an ordered collection of numerical values, e.g., a vector, matrix, or other tensor of numerical values.

"Conditioning" a model (e.g., a generative model) or a neural network (e.g., a denoising neural network) or an operation (e.g., a self-attention operation) on conditioning data (e.g., an embedding representing a protein and one or more ligands) can refer to providing the conditioning data as an input (e.g., a side input) to the model, neural network, or operation, such that outputs generated by the model, neural network, or operation are influenced by (depend on) the conditioning data.

A "binding affinity" of a ligand for a protein refers to the strength or degree of attraction between the ligand and the protein when they interact to form a complex. Binding affinities can be determined experimentally, e.g., by various different assays.

A 3D spatial position of an atom can be represented by a set of coordinates in an appropriate coordinate system, e.g., a 3D Cartesian coordinate system or a spherical coordinate system.

A "structural motif" in a molecule refers to a specific combination and arrangement of atoms in a molecule, and a set of possible structural motifs can include one or more of: aromatic rings, chelating groups, sulfonamides, and so forth. A structural motif can correspond to a defined chemical entity (e.g., ring or group), but is not required to. For instance, a set of possible structural motifs can be generated by performing a statistical analysis to identify the most commonly occurring atomic substructures occurring in molecules in a database of molecules.

A joint 3D structure of a protein and a ligand can define a respective predicted three-dimensional spatial location of each atom in the protein and of each atom in the ligand.

Particular embodiments of the subject matter described in this specification can be implemented so as to realize one or more of the following advantages.

Drug discovery can involve identifying specific molecules within the body that are involved in a disease process. These molecules are often proteins, such as enzymes, receptors, or signaling proteins, that play a key role in the disease's development or progression. A ligand, often a small molecule, peptide, or antibody, can be selected to bind specifically to an identified target protein and modify its biological activity. When a drug that includes the ligand is administered to a patient, the ligand can bind to the target protein with high affinity and in doing so contribute to achieving a therapeutic effect in the patient. For instance, if the target protein is an enzyme involved in a disease process, the ligand can inhibit its activity, thus disrupting the disease pathway. More generally, the interaction between the ligand and the target protein can activate, inhibit, or alter the function of the target protein to achieve a therapeutic effect. Therefore, identifying ligands with high binding affinity for proteins can be a crucial step in the process of drug discovery.

Traditionally, properties of ligands and proteins are evaluated by computational methods such as molecular docking, which can be used to evaluate the binding affinity of a ligand for a protein. Molecular docking of a protein and a ligand involves obtaining data defining respective 3D structures of the protein and the ligand, and performing a search through a space of possible poses of the protein and the ligand to optimize a scoring function. The scoring function can measure, e.g., the energy of each joint conformation of the protein and the ligand. The binding affinity of the ligand for the protein can be derived from the joint pose of the protein and the ligand that optimizes the scoring function.

Conventional methods for predicting protein-ligand properties can be computationally expensive. For instance, in molecular docking, optimizing the scoring function requires searching a large space of possible poses of the protein and the ligand. Moreover, conventional molecular docking requires advance knowledge of the individual 3D structures of the protein and the ligand, and of the binding site(s) on the protein. Further, even if the 3D structure of the protein is known, e.g., from crystallography, the 3D structure of the protein may deform through a process of protein conformational change as the ligand interacts with the protein, e.g., to bind to a binding site on the protein. However, the process of conventional molecular docking does not account for potential conformational changes of the protein as a result of interaction with the ligand which can lead to inaccurate results.

The system described in this specification can predict a property of a protein and a ligand by processing data characterizing the protein and the ligand using an embedding neural network to generate a protein-ligand embedding that jointly represents the protein and the ligand. The system can then process the protein-ligand embedding to generate a predicted property score characterizing a property of the protein and the ligand. The system jointly trains the embedding neural network along with a generative model that, when conditioned on a protein-ligand embedding, can generate a predicted joint three-dimensional (3D) structure of a complex that includes the protein and the ligand represented by the protein-ligand embedding. The task of predicting a property of a protein and a ligand is closely related to the task predicting the joint 3D structure of a complex that includes the protein and the ligand. For instance, the property of binding affinity relates to the energy of the complex formed when the ligand is bound to a binding site on the protein. Jointly training the embedding neural network along with the generative model causes the embedding neural network to generate protein-ligand embeddings that encode rich informational content related to the prediction of 3D structures of protein-ligand complexes, and by extension, related to predicting properties of proteins and ligands.

The prediction system described in this specification overcomes many of the disadvantages of traditional approaches for predicting protein-ligand properties. For instance, the prediction system can generate a predicted protein-ligand property by a single forward pass through a set of machine-learned operations. In contrast, some conventional approaches such as molecular docking require iteratively optimizing a scoring function over a large number (e.g., many thousands) of iterations, which can require significantly more computational resources (e.g., memory and computing power) than the machine-learned operations of the prediction system. Further, in contrast to conventional systems, the prediction system does not require advance knowledge of the 3D structure of the protein, or the 3D structure of the ligand, or even the location of the binding site on the protein. The prediction system is thus more broadly applicable than conventional approaches and can achieve greater accuracy in predicting properties, e.g., because the prediction system can learn to implicitly account for conformational changes in the protein and the ligand during binding.

In some implementations, the prediction system can process a protein-ligand embedding that is generated by an embedding neural network and that jointly represents a protein and a ligand using a property prediction neural network to generate a predicted property of the protein and the ligand. The protein-ligand embedding can encode rich informational content related to the joint 3D structure of the protein and the ligand (as a result of being jointly trained with the generative model, as described above), and the property prediction neural network can be trained to leverage this rich informational content to accurately predict the protein-ligand property.

In some implementations, the prediction system can condition the generative model on the protein-ligand embedding generated by the embedding neural network, and then generate a predicted 3D structure of the protein-ligand complex using the generative model. The system can generate a graph representing (at least a portion of) the 3D structure of the protein-ligand complex, and process the graph using a graph neural network to generate a predicted protein-ligand property. The system can accurately and efficiently predict the protein-ligand property by leveraging an efficient graph representation of the 3D structure of the protein-ligand complex and by using a machine learning model (in particular: a graph neural network) that is specially configured to operate on graph-structured data. Optionally, as part of generating the graph representing the 3D structure of the protein-ligand complex, the system can include "super nodes" in the graph that represent higher-level structures such as amino acids or ligand structural motifs and that facilitate efficient information propagation across the graph during processing by the graph neural network, as will be described in more detail below.

In some implementations, the prediction system implements the generative model as a generative diffusion model that, when conditioned on the protein-ligand embedding, can predict the 3D structure of a protein-ligand complex by iteratively denoising the 3D spatial positions of the atoms in the complex. The prediction system can augment the set of data being denoised by the generative diffusion model to include data defining the protein-ligand property, i.e., in addition to the 3D spatial positions of the atoms in the complex. The generative diffusion model can thus jointly predict the protein-ligand property along with the 3D structure of the complex in a manner that iteratively improves the accuracy of the predicted property by gradually incorporating and leveraging 3D structure data characterizing the protein-ligand complex.

The details of one or more embodiments of the subject matter of this specification are set forth in the accompanying drawings and the description below. Other features, aspects, and advantages of the subject matter will become apparent from the description, the drawings, and the claims.

BRIEF DESCRIPTION OF THE DRAWINGS

FIG. 1A shows an example property prediction system.
FIG. 1B shows an example embedding neural network.
FIG. 1C is a flow diagram of an example process for processing a protein embedding of a protein and a ligand embedding of a ligand using a fusion neural network to generate a protein - ligand embedding that jointly represents the protein and the ligand.
FIG. 1D illustrates operations performed by the property prediction system to generate the protein - ligand embedding.
FIG. 2 is a flow diagram of an example process for generating a predicted property of the protein and the ligand using a property prediction neural network.
FIG. 3 is a flow diagram of an example process for jointly training a property prediction neural network with the embedding neural network of the property prediction system.
FIG. 4A is a flow diagram of an example process for generating a predicted property score for a protein and a ligand using a graph neural network.
FIG. 4B is a flow diagram of an example process for generating a graph based on a predicted 3D structure of a protein-ligand complex.
FIG. 4C is a flow diagram of an example process for processing a graph representing a 3D structure of a protein-ligand complex using a graph neural network to generate a predicted property score.
FIG. 5 is a flow diagram of an example process for training a graph neural network.
FIG. 6 is a flow diagram of an example process for generating a predicted joint 3D structure of a protein and a ligand and, optionally, a predicted property of the protein and the ligand, using a generative diffusion model that includes a denoising neural network.
FIG. 7A is a flow diagram of an example process for generating a denoising output using a denoising neural network.
FIG. 7B is a flow diagram of an example process for updating a set of current atom embeddings using a self-attention operation that is implemented by a self-attention block of the denoising neural network and that is conditioned on a protein - ligand embedding.
FIG. 8 is a flow diagram of an example process for jointly training the embedding neural network and the generative model of the property prediction system.
FIG. 9 is a flow diagram of an example process for jointly training an embedding neural network and a generative diffusion model on a training example.

Like reference numbers and designations in the various drawings indicate like elements.

DETAILED DESCRIPTION

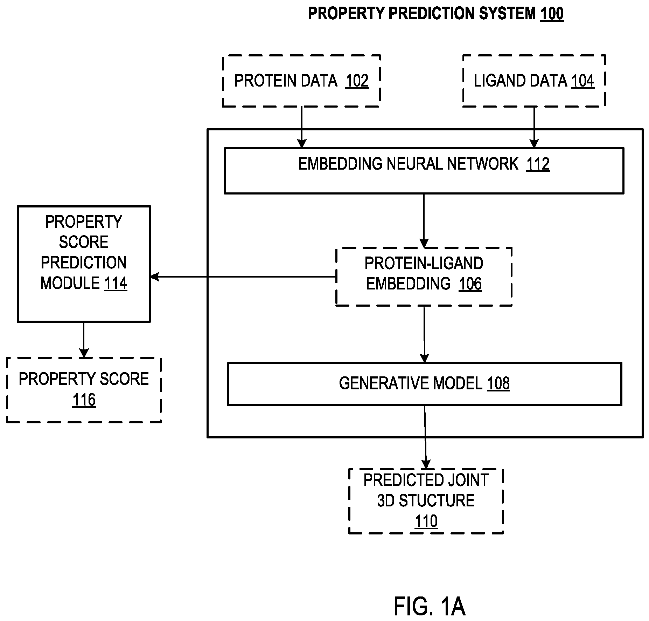

FIG. 1A shows an example property prediction system 100. The property prediction system 100 is an example of a system implemented as computer programs on one or more computers in one or more locations in which the systems, components, and techniques described below are implemented.

The property prediction system 100 is configured process data characterizing a protein 102 ("protein data") and data characterizing a ligand 104 ("ligand data") to generate a property score 116 that defines a predicted property of the protein and the ligand.

The property score 116 can characterize any appropriate property of the protein and the ligand. A few examples of possible property scores are described next.

In one example, the property score can define a likelihood of occurrence of a binding event that involves the protein and the ligand.

In another example, the property score can define a binding affinity of the ligand and the protein, e.g., as measured using a particular binding affinity assay, as will be described in more detail below. The binding affinity of the ligand and the protein characterizes a strength or degree of attraction between the ligand and the protein when they interact to form a complex.

In another example, the property score can define a likelihood that the ligand is an agonist for the protein. For example, the property score can define a likelihood that the ligand is an agonist of a receptor that includes the protein, i.e., binds to the protein to activate the receptor to produce a biological response.

In another example, the property score can define a likelihood that the ligand is an antagonist for the protein. For example, the property score can define a likelihood that the ligand is an antagonist of a receptor that includes the protein, e.g., binds to the protein to prevent activation of the receptor to dampen or inhibit a biological response

In another example, the property score can characterize any appropriate predicted downstream effect of the ligand acting on the protein. For instance, the property score can define a predicted potency of the ligand in acting on the protein, e.g., as measured by a half maximal effective concentration (EC50) of the ligand when acting on the protein. In another example, the property score can define a predicted inhibitory effect of the ligand when acting on the protein, e.g., as measured by a half maximal inhibitory concentration (IC50) of the ligand when acting on the protein.

Optionally, the system can generate multiple property scores, i.e., instead of a single property score. For instance, the system can generate any combination of two or more of the example property scores that are described above.

The protein data 102 can include any appropriate data characterizing the protein, e.g., data defining one or more amino acid sequences of the protein, or data defining an MSA for the protein, or data characterizing a respective structure of each of one or more "template" proteins, or a combination thereof. A template protein can refer to a protein that is "similar" to the protein 102, e.g., such that the value of a similarity measure between the template protein and the protein 102 satisfies (e.g., exceeds) a threshold (e.g., 0.8, or 0.9, or 0.99, or any other appropriate threshold). Similarity between a first protein and a second protein can be measured using any appropriate similarity measure, e.g., a sequence identity or percent identity similarity measure between the respective amino acid sequence(s) of the first protein and the second protein. The structure of a template protein can be represented in any appropriate manner, e.g., by a contact map, or by data defining a respective 3D spatial position of each atom in the template protein. Optionally, the protein data 102 can exclude any data that directly defines the 3D structure of the protein, e.g., the 3D spatial locations of the atoms or amino acid residues in a 3D conformation of the protein.

The ligand data 104 can include any appropriate data characterizing a ligand. A ligand can refer to a molecule or compound that binds to a target molecule, e.g., a protein. Ligands can include, e.g., small organic molecules, complex organic molecules, proteins, biomolecules (e.g., polynucleotides or polypeptides), and so forth. For instance, the ligand data 104 can include a textual representation of one or more of: a chemical structure of the ligand (e.g., the arrangement of atoms and bonds in the ligand), the atom types in the ligand and their connectivity, the chirality of the bonds in the ligand, or any functional groups (e.g., hydroxyl groups, amino groups, carboxyl groups, and so forth) included in the ligand. The textual representation of the ligand can include, e.g., a simplified molecular-input line-entry system (SMILES) string characterizing the ligand. As another example, the ligand data 104 can include a representation of the ligand by way of graph data representing a graph, e.g., where the nodes in the graph represent atoms in the ligand and the edges in the graph represent bonds between atoms in the ligand. Optionally, the ligand data 104 can exclude any data that directly defines the 3D structure of each ligand, e.g., the 3D spatial locations of the atoms in a 3D conformation of the ligand.

The property prediction system 100 processes the protein data 102 and the ligand data 104 using an embedding neural network 112. The embedding neural network 112 is configured to process the protein data 102 and the ligand data 104 to generate a protein - ligand embedding 106 that jointly represents the protein and the ligand.

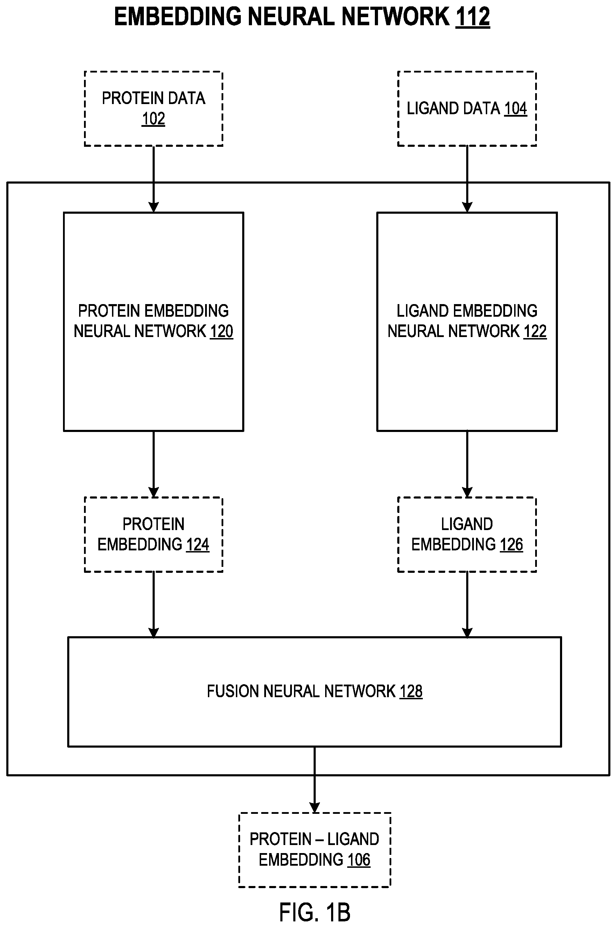

The embedding neural network 112 can have any appropriate neural network architecture that enables the embedding neural network 112 to perform its described functions. In particular, the embedding neural network 112 can include any appropriate types of neural network layers (e.g., fully connected layers, convolutional layers, attention layers, etc.) in any appropriate number (e.g., 5 layers, 10 layers, or 20 layers) and connected in any appropriate configuration (e.g., as a directed graph of layers). An example architecture of the embedding neural network is described in more detail with reference to FIG. 1B.

The embedding neural network 112 can be jointly trained with a generative model 108. The generative model 108, when conditioned on the protein - ligand embedding 106, is configured to generate one or more predicted joint 3D structures 110 of the complex. The predicted joint 3D structure 110 defines a respective predicted 3D spatial location of each atom in the protein and ligand, i.e., of each atom in the protein and of each atom in the ligand. The predicted joint 3D structure can define a structure of the complex where the ligand is bound to a binding site on the protein.

The generative model 108 can be any appropriate conditional generative model. More specifically, the generative model 108 can any appropriate model that, when conditioned on the protein - ligand embedding 106, can generate samples from a distribution over a space of possible joint 3D structures of the complex. For instance, the generative model 108 can be implemented as a generative diffusion model, or a generative adversarial neural network (GAN) model, or a flow-based neural network model (normalizing flow model), and so forth.

Optionally, the generative model 108 can generate multiple distinct predicted joint 3D structures of the complex. In particular, the generative model 108 can generate multiple samples from the distribution over the space of possible joint 3D structures of the complex. Differences between the predicted joint 3D structures generated by the generative model 108 can reflect both uncertainty in the predicted structure and also the various structural modes (e.g., different conformations) of a complex that includes the protein and the ligand.

The property prediction system 100 can jointly train the embedding neural network 112 and the generative model 108 on a set of training data using an appropriate machine learning training technique. The training data can include a set of training examples, where each training example corresponds to a complex of a protein and a ligand, e.g., where the ligand is bound to a binding site on the protein. Each training example can include (i) a training input that characterizes a training protein and a training ligand, and (ii) a target output based on a joint 3D structure of the training protein and the training ligand.

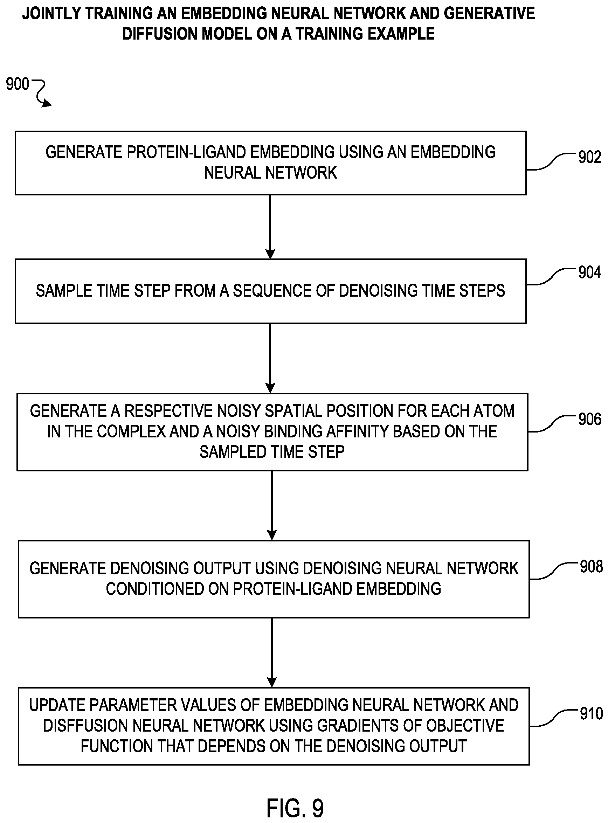

For example, the machine learning training technique can include processing the training input of the training example using the embedding neural network to generate protein-ligand embedding of the training protein and the training ligand. The property prediction system can then process the protein-ligand embedding of the training protein and the training ligand using the generative model to generate a predicted output characterizing a predicted joint 3D structure of the training protein and the training ligand of the training example. The property prediction system 100 can backpropagate gradients of an objective function through the generative model and into the embedding neural network. For example, gradients of the objective function with respect to parameters of the generative model and gradients of the objective function with respect to parameters of the embedding neural network can be determined by backpropagation, and the gradients used to adjust values of the parameters of the embedding neural network and the embedding neural network to optimize the objective function. The objective function can measure a discrepancy between the target output specified by the training example and the predicted output generated by the embedding neural network and the generative model for the training example. An example process for jointly training the embedding neural network and a generative diffusion model (parametrized by a denoising neural network) is described in more detail with reference to FIG. 9.

The property prediction system 100 can use a property score prediction module 114 to generate a property score 116 that defines a predicted property of the protein and the ligand using the protein-ligand embedding 106.

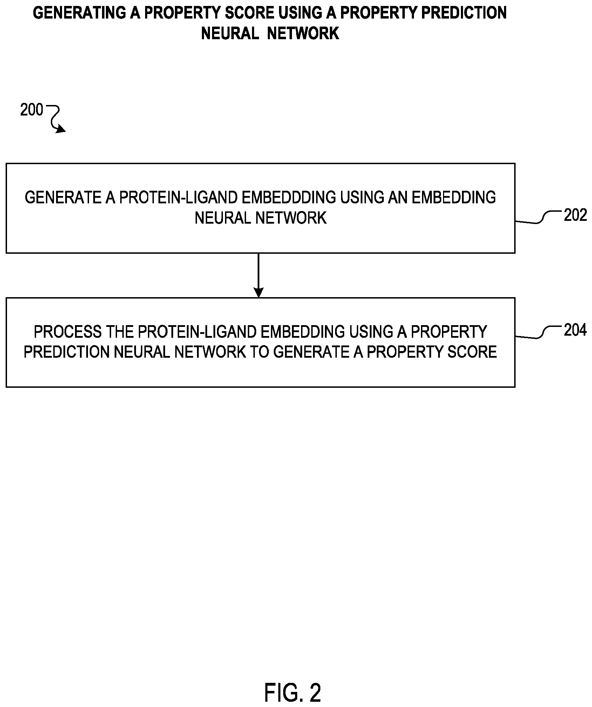

In some implementations, the property prediction system 100 generates the predicted property 116 by processing the protein-ligand embedding 106 using a property prediction neural network. These implementations are described in more detail with reference to FIG. 2 - FIG. 3.

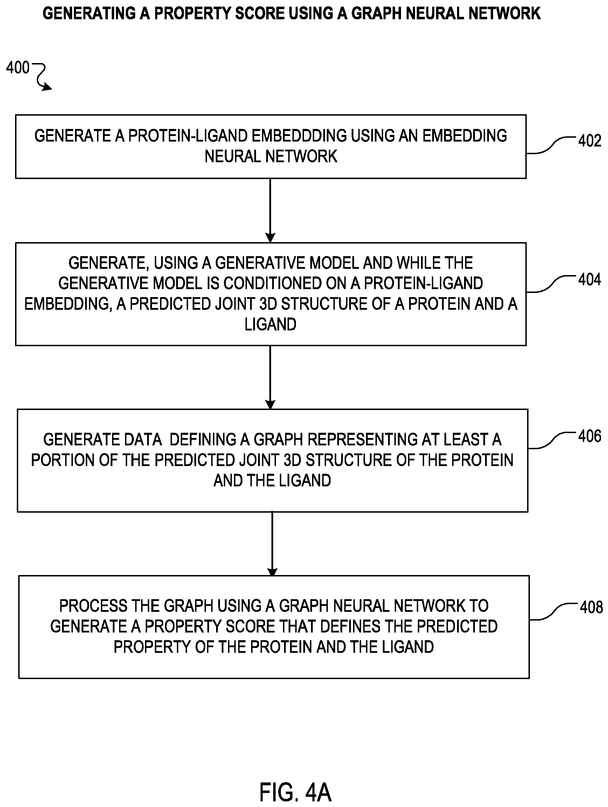

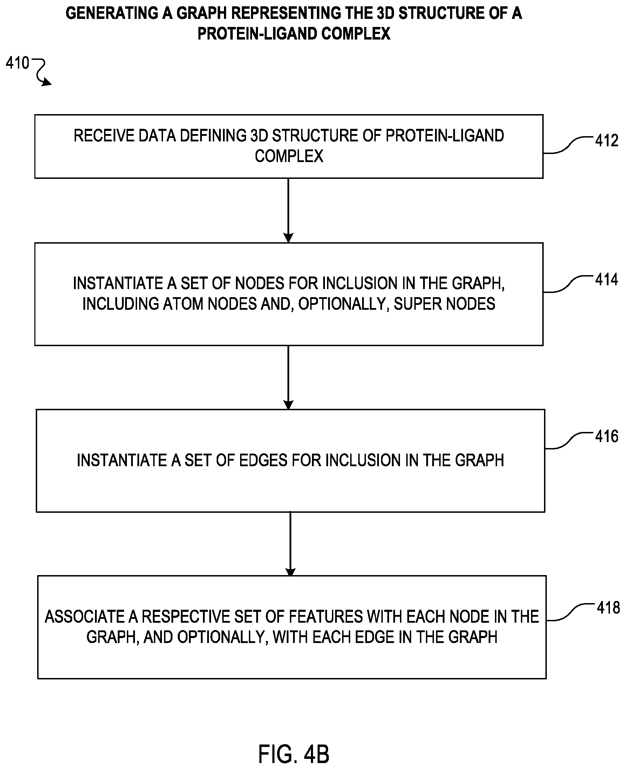

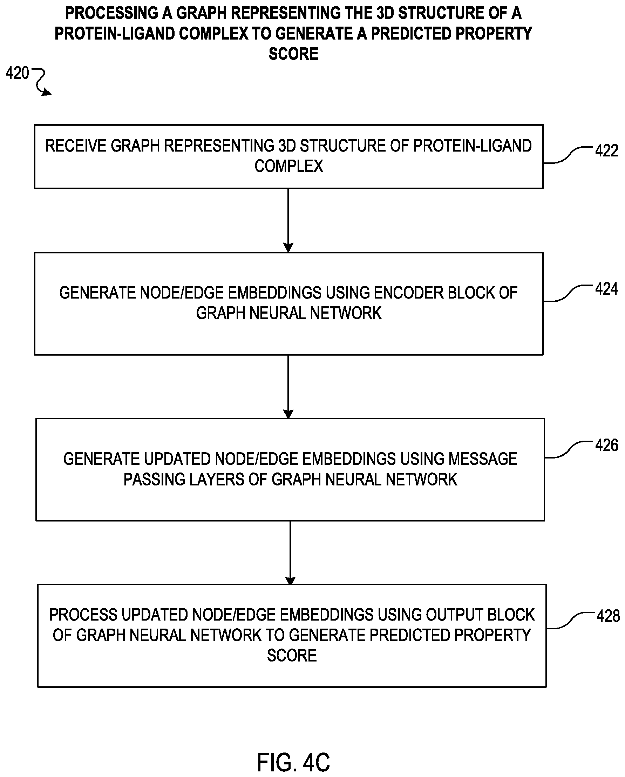

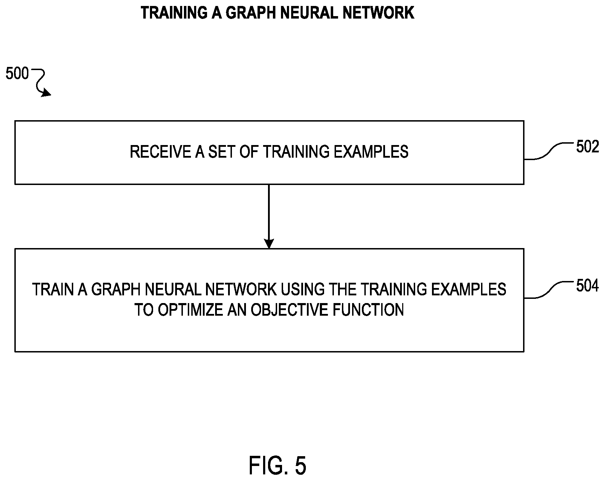

In some implementations, the property prediction system 100 generates the predicted property 116 by generating a predicted 3D structure of the complex using the generative model, generating a graph representation of the 3D structure of the complex, and then processing the graph representation using a graph neural network. These implementations are described in more detail with reference to FIG. 4A, FIG. 4B, Fig. 4C, and FIG. 5.

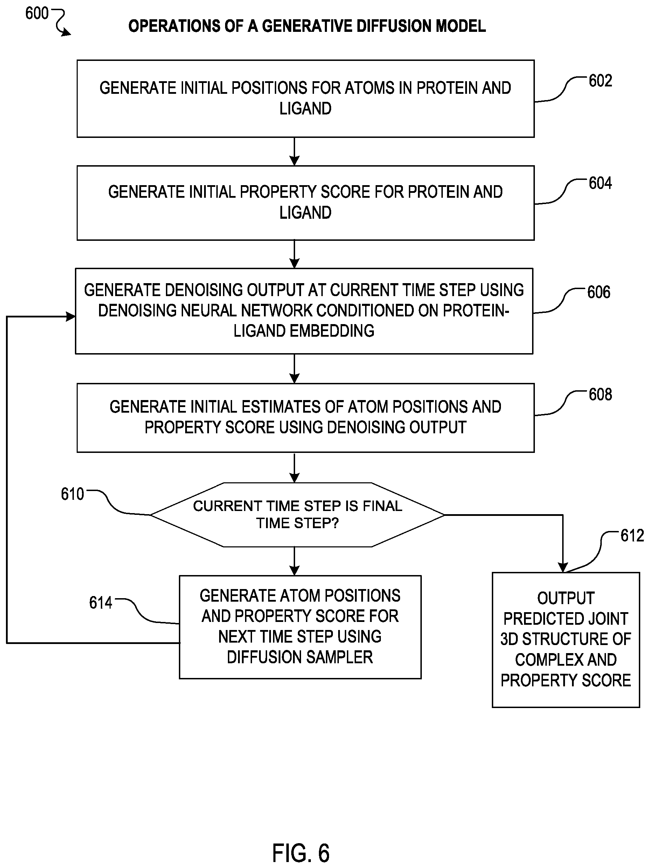

In some implementations, the generative model 108 is implemented as a generative diffusion model, and the property prediction system 100 generates the predicted property 116 by iteratively denoising the 3D spatial positions of the atoms in the complex along with data defining the protein-ligand property. These implementations are described in more detail with reference to FIG. 6 - FIG. 9.

In some examples, the property score prediction module 114 generates a binding affinity score that defines the predicted binding affinity of the protein and the ligand by conditioning the generation of the binding affinity score on data specifying a type of binding affinity assay. The binding affinity score can define the predicted binding affinity of the protein and the ligand as measured by the specified type of binding affinity assay. The binding affinity score can correspond to any appropriate type of binding affinity assay, i.e., any appropriate experimental technique for quantitatively measuring binding affinity. The type of binding affinity assay can be, for example, a surface plasmon resonance (SPR) assay, or an isothermal titration calorimetry (ITC) assay, or a fluorescence-based assay (e.g., a fluorescence-based polarization (FP) assay), or an enzyme-linked immunosorbent assay (ELISA), or a radioligand binding assay, or a bioluminescence resonance energy transfer (BERT) assay, etc. In some implementations, a user of the system can specify the type of assay corresponding to the binding affinity to be generated by the system, e.g., by way of a user interface or application programming interface (API) made available by the system. Example mechanisms by which the system can condition the generation of the binding affinity score on data specifying a type of binding affinity assay are described in more detail below.

Providing a mechanism for conditioning on the type of binding affinity assay can increase the amount of training data available for training the system, e.g., because the system can be trained on binding affinity training data associated with a variety of different binding affinity assays rather than being limited to only training data associated with a single binding affinity assay.

The property prediction system 100 can receive the protein data 102 and the ligand data 104 from any appropriate source, e.g., from a user or from another system, by way of an appropriate interface, e.g., an application programming interface (API) or a user interface (e.g., a graphical user interface). After generating the predicted property score 116, the property prediction system 100 can, e.g., store data defining the predicted property score 116 in a memory or transmit data defining the predicted property score 116 over a data communication network or provide data defining the predicted property score 116 directly to a system that performs downstream processing based on the predicted property score 116.

Predicted property scores 116 generated by the property prediction system 100 can be used in any of a variety of possible downstream applications. A few examples of downstream applications that process predicted property scores 116 generated by the property prediction system 100 are described next.

In some cases, predicted property scores 116 generated by the property prediction system 116 can be used for drug discovery. Drug discovery can involve identifying specific molecules within the body that are involved in a human or animal disease process. These molecules are often proteins, such as enzymes, receptors, or signaling proteins, that play a key role in the disease's development or progression. A ligand, often a small molecule, peptide, antibody, or aptamer, can be selected to bind specifically to an identified target protein. When a drug that includes the ligand is administered to a patient, the ligand can bind to the target protein with high affinity and in doing so contribute to achieving a therapeutic effect in the patient. For instance, if the target protein is an enzyme involved in a disease process, the ligand can inhibit its activity, thus disrupting the disease pathway. More generally, the interaction between the ligand and the target protein can activate, inhibit, or alter the function of the target protein to achieve a therapeutic effect.

Therefore, identifying ligands with high (or low) binding affinity for a protein can be a crucial step in the process of drug discovery. (Identifying ligands with low binding affinities for a protein can be desirable, e.g., when the protein is an off-target protein and the binding of the ligand to the protein may cause undesirable side effects). However, determining binding affinities of ligands for proteins, e.g., through computational simulations or physical experiments, can be expensive and time consuming.

To address these issues, predicted property scores 116 generated by the property prediction system 100 can be used to determine a ranking of candidate ligands in a collection of candidate ligands based on their respective predicted property scores, e.g., binding affinities for a protein. More specifically, for each candidate ligand, the property prediction system 100 can generate a respective predicted property score 116 of the candidate ligand for the protein. The collection of candidate ligands can then be ranked based on the respective predicted property score of each candidate ligand.

The ranking of the candidate ligands in the collection of candidate ligands based on their respective predicted property scores can be used, e.g., to select a proper subset of the collection of candidate ligands for experimental validation and testing. For instance, one or more candidate ligands having the highest or lowest predicted property scores (i.e., according to the ranking) can be selected for experimental validation and testing, e.g., for use in a drug that achieves a therapeutic effect in patients. In particular, each selected candidate ligand can be physically synthesized and then tested, e.g., in vitro (such as in a cell culture or tissue model) or in vivo, for a variety of properties, e.g., absorption, distribution, metabolism, and/or excretion, by a living organism or cell culture or tissue model. For example, the selected candidate ligand(s) can be screened according to a degree to which binding is accompanied by a biological (therapeutic) effect such as facilitating a biological mechanism or directly or indirectly inhibiting a biological disease mechanism (e.g. inhibiting a bacteria or virus from entering a cell), toxicity, clearance time, and so forth. One or more of the candidate ligands from the collection of ligands can be selected for inclusion in a drug, e.g., based at least in part on results of the testing. A drug that includes one or more of the candidate ligands can be synthesized using any appropriate drug synthesis technique.

As another example, the target protein molecule may be an enzyme comprising a CRISPR associated protein and the ligand may comprise a guide RNA molecule. The system can, for example, identify a combination of guide RNA molecule and CRISPR associated protein, in particular one that operates efficiently to edit genes. Such a method can involve determining that the enzyme and the guide RNA fit and work together effectively. The guide RNA may have a part with a defined 3D structure, e.g., it may be a single guide RNA (sgRNA), incorporating a guide sequence and a tracrRNA sequence.

As another example, the system can be used to identify a drug which is able to disrupt a protein complex or inhibit formation of the complex. Some diseases, e.g., neurodegenerative diseases such as dementia, are caused by protein aggregation. The system may thus be used to identify a ligand that is a drug to treat such a disease.

The collection of candidate ligands can include any appropriate number of ligands, e.g., 10 ligands, or 1000 ligands, or 100,000 ligands. In some cases, only a fraction of the candidate ligands in the set of candidate ligands are selected for physical synthesis, e.g., based on the ranking of the candidate ligands by their predicted property scores. For instance, less than 50%, or less than 10%, or less than 1%, or less than 0.1% of the candidate ligands in the collection of candidate ligands may be selected for physical synthesis.

The candidate ligand(s) may be derived from a database of candidate ligands, and/or may be derived by modifying ligands in a database of candidate ligands, e.g., by modifying a structure or amino acid sequence of a candidate ligand, and/or may be derived by stepwise or iterative assembly/optimization of a candidate ligand. The candidate ligand(s) may alternately or additionally include one or more candidate ligands generated using a generative model conditioned on (the structure of) the target protein molecule or part of the target protein molecule, e.g., a structure of a binding site or other part of the target protein molecule.

In some implementations the candidate ligand(s) may include small molecule complex ligands, e.g., organic compounds with a molecular weight of <900 daltons. In some other implementations the candidate ligand(s) may include polypeptide ligands, i.e., defined by an amino acid sequence.

In some implementations a candidate (e.g. polypeptide or polynucleotide) ligand may include: an isolated antibody or aptamer, a fragment of an isolated antibody or aptamer, a single variable domain antibody, a bi- or multi-specific antibody, a multivalent antibody, a dual variable domain antibody, an immuno-conjugate, a fibronectin molecule, an adnectin, an DARPin, an avimer, an affibody, an anticalin, an affilin, a protein epitope mimetic or combinations thereof. A candidate (polypeptide) ligand may include an antibody with a mutated or chemically modified amino acid Fc region, e.g., which prevents or decreases ADCC (antibody-dependent cellular cytotoxicity) activity and/or increases half-life when compared with a wild type Fc region. Candidate (polypeptide or polynucleotide) ligands may include antibodies with different CDRs (Complementarity-Determining Regions).

In some implementations, one or more selected ligands can by synthesized, i.e., by making, the small molecule, polynucleotide or polypeptide ligand. The one or more ligands may be synthesized by any conventional chemical techniques (including e.g., biosynthetic techniques) and/or may already be available, e.g., may be from a compound library or may have been synthesized using combinatorial chemistry.

One or more selected ligands can be tested for biological activity in vitro and/or in vivo. For example each selected ligand may be tested for ADME (absorption, distribution, metabolism, excretion) and/or toxicological properties, to screen out unsuitable ligands. The testing may include, e.g., bringing the candidate small molecule, polypeptide or polynucleotide ligand into contact with the target protein molecule and measuring a change in expression or activity of the target molecule.

In some cases, predicted property scores 116 generated by the property prediction system 100 can be used for drug repurposing. In more detail, an existing drug may include a particular ligand, e.g., that is known to achieve a therapeutic effect in patients by binding to a target protein involved with a particular disease process. Drug repurposing can involve identifying new protein binding targets for the ligand, e.g., that are potentially involved in different disease processes. If the ligand has a high binding affinity (or potency, or inhibitory effect, etc.) for a new target protein that is involved in a disease process, then the ligand can be selected for experimental validation and potential inclusion in a drug for treating the disease. Drug repurposing can leverage the safety and efficacy data already available for a drug that includes the ligand, potentially accelerating the development process and reducing research costs. Drug repurposing can identify novel treatment options and address unmet medical needs by repurposing known ligands to treat different diseases or conditions.

To identify new target proteins for a ligand, predicted property scores 116 generated by the property prediction system 100 can be used to determine a ranking of candidate proteins in a collection of candidate proteins based on a respective predicted property score of a particular ligand relative to each of the candidate proteins. More specifically, for each candidate protein, the property prediction system 100 can generate a respective predicted property score for the ligand and the candidate protein. The collection of candidate proteins can then be ranked based on the respective predicted property score of the ligand relative to each of the candidate proteins.

The ranking of the candidate proteins in the collection of candidate proteins based on the predicted property scores of the ligand relative to the candidate proteins can be used, e.g., to select a proper subset of the collection of candidate proteins for experimental validation and testing. For instance, one or more candidate proteins for which the ligand has the highest predicted property score (i.e., according to the ranking) can be selected for experimental validation and testing, e.g., for use in a drug that achieves a therapeutic effect in patients. In particular, each selected candidate protein can be physically synthesized and the properties of the ligand relative to the candidate protein can then be experimentally tested and validated. One or more of the candidate proteins from the collection of candidate proteins can be selected as binding targets for the ligand, e.g., based at least in part on results of the testing.

The collection of candidate proteins can include any appropriate number of proteins, e.g., 10 proteins, or 1000 proteins, or 100,000 proteins. In some cases, only a fraction of the candidate proteins in the set of candidate proteins are selected for physical synthesis, e.g., based on the ranking of the candidate proteins by the predicted property score of the ligand relative to the candidate proteins. For instance, less than 50%, or less than 10%, or less than 1%, or less than 0.1% of the candidate proteins in the collection of candidate proteins may be selected for physical synthesis.

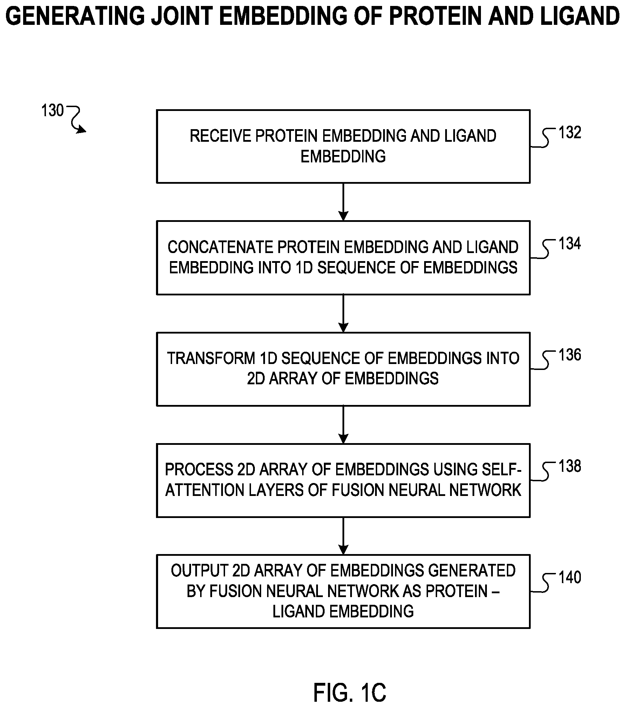

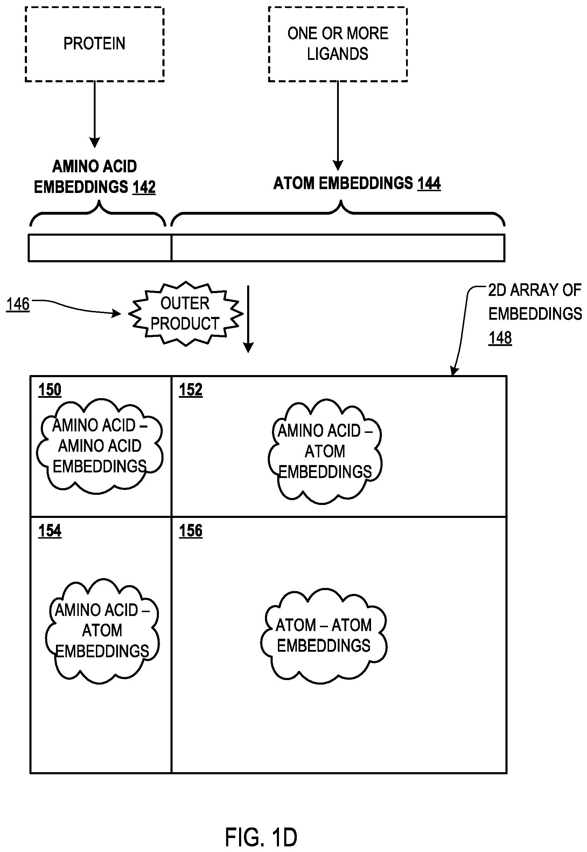

FIG. 1B shows an example embedding neural network 112, e.g., that is included in the property prediction system described with reference to FIG. 1. The embedding neural network 112 is configured to process: (i) protein data 102 characterizing a protein, and (ii) ligand data 104 characterizing a ligand, to generate a protein - ligand embedding 106 of the protein and the ligand.

The embedding neural network 112 includes a protein embedding neural network 112, a ligand embedding neural network 112, and a fusion neural network 128, which are each described in more detail next (and throughout this specification).

The protein embedding neural network 112 is configured to process the protein data 102 characterizing the protein to generate a protein embedding 124 of the protein. The protein embedding 124 can include, e.g., a respective amino acid embedding of each amino acid in each amino acid sequence of the protein.

The protein embedding neural network 112 can have any appropriate neural network architecture that enables the protein embedding neural network 112 to perform its described functions. In particular, the protein embedding neural network 112 can include any appropriate types of neural network layers (e.g., fully connected layers, convolutional layers, attention layers, etc.) in any appropriate number (e.g., 5 layers, 10 layers, or 20 layers) and connected in any appropriate configuration (e.g., as a directed graph of layers).

A particular example of a possible architecture of the protein embedding neural network 112 is the "Evoformer" neural network described in Jumper et al., "Highly accurate protein structure prediction with AlphaFold," Nature, Vol 596, 26 August 2021. The Evoformer can process a network input derived from: (i) the amino acid sequence of a protein, (ii) an MSA for the protein, and (iii) the 3D structures of one or more template amino acid sequences, to generate an output that includes a "single representation" that defines a respective embedding of each position in each amino acid sequence of the protein. (A template amino acid sequence is an MSA sequence for an amino acid chain in the protein where the folded structure of the template sequence is known, e.g., from physical experiments).

The ligand embedding neural network 112 is configured to process the ligand data 104 characterizing the ligand to generate a ligand embedding 126 of the ligand. The embedding of the ligand can include a respective atom embedding representing each atom in the ligand.

The ligand embedding neural network 112 can have any appropriate neural network architecture that enables the ligand embedding neural network 112 to perform its described functions. In particular, the ligand embedding neural network 112 can include any appropriate types of neural network layers (e.g., fully connected layers, convolutional layers, attention layers, etc.) in any appropriate number (e.g., 5 layers, 10 layers, or 20 layers) and connected in any appropriate configuration (e.g., as a directed graph of layers).

In a particular example, the ligand embedding neural network 112 can be configured to receive a collection of initial atom embeddings that includes a respective initial atom embedding for each atom in the ligand and that is derived from data characterizing the ligand, e.g., a SMILES string representing the ligand. The initial atom embedding of each atom can include data characterizing the type of the atom, the other atoms to which the atom is bonded, whether the atom is included in any functional groups, and so forth. The ligand embedding neural network can process the collection of initial atom embeddings by a sequence of one or more attention neural network layers, that are each configured to update the collection of current atom embeddings by a self-attention operation, to generate the embedding of the ligand, e.g., as the collection of atom embeddings output by a final attention layer in the sequence of attention layers.

The fusion neural network 128 is configured to process the protein embedding 124 and the ligand embedding 126 to generate the protein - ligand embedding 106 that jointly represents the protein and the ligand. The fusion neural network 128 can have any appropriate neural network architecture that enables the fusion neural network 128 to perform its described functions. In particular, the fusion neural network 128 can include any appropriate types of neural network layers (e.g., fully connected layers, convolutional layers, attention layers, etc.) in any appropriate number (e.g., 5 layers, 10 layers, or 20 layers) and connected in any appropriate configuration (e.g., as a directed graph of layers). An example of processing a protein embedding 124 and a ligand embedding 126 to generate a protein - ligand embedding 106 is described in more detail with reference to FIG. 1C.

FIG. 1C is a flow diagram of an example process 130 for processing a protein embedding of a protein and a ligand embedding of a ligand using a fusion neural network to generate a protein - ligand embedding that jointly represents the protein and the ligand. For convenience, the process 130 will be described as being performed by a system of one or more computers located in one or more locations. For example, a property prediction system, e.g., the property prediction system 100 of FIG. 1, appropriately programmed in accordance with this specification, can perform the process 130.

The system receives a protein embedding of a protein and a ligand embedding of a ligand (132). The protein embedding can be generated by a protein embedding neural network and can include a respective amino acid embedding for each position in each amino acid sequence of the protein. The ligand embedding can be generated by a ligand embedding neural network and can include a respective atom embedding for each atom in the ligand.

The system concatenates the protein embedding and the ligand embedding to generate a one-dimensional (1D) sequence of embeddings (134). The 1D sequence of embedding includes the amino acid embeddings of the protein embedding and the atom embeddings of the ligand embedding. The embeddings included in the 1D sequence of embeddings can be ordered in any appropriate way, e.g., the 1D sequence of embeddings can be ordered to have the amino acid embeddings followed by the atom embeddings, or to have the atom embeddings followed by the amino acid embeddings. The length of the 1D sequence of embeddings can be a sum of: (i) the number of amino acid embeddings in the protein embedding, and (ii) the number of atom embeddings in the ligand embedding. The 1D sequence of embedding can be represented by data having dimensionality $NumTokens \times d$, where $NumTokens$ is given by the sum of: (i) the number of amino acid embeddings in the protein embedding, and (ii) the number of atom embeddings in the ligand embedding, and $d$ is a positive integer value defining the number of channel dimensions in each amino acid embedding and atom embedding.

The system processes the 1D sequence of amino acid embeddings and atom embeddings to generate data defining a two-dimensional (2D) array of embeddings (136). The 2D array of embeddings can be represented by data having dimensionality $NumTokens \times NumTokens \times d'$ where (as above) $NumTokens$ is given by the sum of: (i) the number of amino acid embeddings in the protein embedding, and (ii) the number of atom embeddings in the ligand embedding, and $d'$ is a positive integer value defining the number of channel dimensions in each embedding ($d'$ can be equal to $d$, i.e., the number of channel dimensions in each amino acid embedding and atom embedding). The system can generate the 2D array of embeddings from the 1D sequence of embeddings in any of a variety of ways. For instance, the system can generate the 2D array of embeddings as a result of an element-wise outer product of the 1D sequence of embeddings with itself. As another example, the system can generate the 2D array by an appropriate 2D concatenation operation, e.g., where the embedding at each position $(i, j)$ in the 2D array of embeddings is generated by concatenating: (i) the embedding at position i, and (ii) the embedding at position $j$, in the 1D sequence of amino acid embeddings (where indices $i, j \in \{1, ... N\}$, where $N$ is the length of the 1D sequence of embeddings).

Each embedding in the 2D array of embeddings can be, e.g.: (i) an atom - atom embedding, or (ii) an amino acid - amino acid embedding, or (iii) an amino acid - atom embedding. Each atom - atom embedding is derived from a pair of atom embeddings representing atoms in the ligand. Each amino acid - amino acid embedding is derived from a pair of amino acid embeddings representing amino acids in the protein. Each amino acid - atom embedding is derived from a pair of embeddings that includes an amino acid embedding representing an amino acid in the protein and an atom embedding representing an atom in the ligand.

The system processes the 2D array of embeddings by a set of neural network layers of the fusion neural network to generate an updated 2D array of embeddings defining the protein - ligand embedding (138). The updated 2D array of embeddings can have the same dimensionality as the original 2D array of embeddings, e.g., $NumTokens \times NumTokens \times d'$ (where $NumTokens$ and $d'$ are defined as above). To generate the updated 2D array of embeddings, the fusion neural network can process the 2D array of embeddings using a sequence of one or more self-attention blocks. Each self-attention block can be configured to receive the current 2D array of embeddings as an input, to update the current 2D array of embeddings by one or more self-attention operations (e.g., single-head or multi-head query-key-value (QKV) self-attention operations), and to provide the updated 2D array of embeddings to a subsequent neural network layer (e.g., to another self-attention block, or to an output layer of the fusion neural network).

The self-attention blocks of the fusion neural network can implement any appropriate self-attention operations. A few examples of self-attention operations that can be implemented by self-attention blocks of the fusion neural network are described next.

In some implementations, one or more of the self-attention blocks of the fusion neural network implement "row-wise" or "column-wise" self-attention over the current 2D array of embeddings (i.e., that is provided as an input to the self-attention block). In a row-wise self-attention operation, a self-attention layer updates each given embedding in the 2D array of embeddings using a self-attention operation over only embeddings located in the same row as the given embedding in the 2D array of embeddings. In a column-wise self-attention operation, a self-attention block updates each given embedding in the 2D array of embeddings using a self-attention operation over only embeddings located in the same column as the given embedding in the 2D array of embeddings.

In some implementations, one or more of the self-attention blocks of the fusion neural network implement triangle self-attention operations. An example implementation of triangle self-attention operations is described in Jumper et al., "Highly accurate protein structure prediction with AlphaFold," Nature, Vol 596, 26 August 2021.

In some implementations, one or more of the self-attention blocks of the fusion neural network implement a full self-attention operation over the current 2D array of embeddings, e.g., by updating each embedding in the 2D array of embeddings using attention over the entire 2D array of embeddings.

The fusion neural network can include other neural network layers, i.e., in addition to the sequence of self-attention blocks, e.g., other neural network layers (such as fully connected layers or normalization layers) that are interleaved among the self-attention blocks. The fusion neural network can also include features such as skip connections, e.g., to implement residual blocks in the fusion neural network.

The system outputs the 2D array of embeddings generated by the fusion neural network as the protein - ligand embedding (140). The system can provide protein - ligand embedding generated by the fusion neural network, e.g., for conditioning the generative model and/or for predicting one or more properties of the protein and the ligand.

FIG. 1D illustrates operations performed by the property prediction system to generate the protein - ligand embedding. The system generates a protein embedding that includes a sequence of amino acid embeddings 142 (one for each amino acid in each amino acid sequence of the protein) and a ligand embedding that includes a sequence of atom embeddings 144 (one for each atom in the ligand). The property prediction system concatenates the sequence of amino acid embeddings and the sequence of atom embeddings into a 1D sequence of embeddings, and then transforms the 1D sequence of embeddings (e.g., by an outer product operation) into a 2D array of embeddings. The 2D array of embeddings can include: (i) atom - atom embeddings 156, (ii) amino acid - amino acid embeddings 150, and (iii) amino acid - atom embeddings 152, 154. Each atom - atom embedding is derived from a pair of atom embeddings representing atoms in the ligand. Each amino acid - amino acid embedding is derived from a pair of amino acid embeddings representing amino acids in the protein. Each amino acid - atom embedding is derived from a pair of embeddings that includes an amino acid embedding representing an amino acid in the protein and an atom embedding representing an atom in the ligand. The property prediction system can process the 2D array of embeddings 148 using a sequence of one or more self-attention blocks (e.g., that implement row-wise attention, or column-wise attention, or triangle self-attention, or full self-attention) to generate the protein - ligand embedding.

FIG. 2 is a flow diagram of an example process 200 for generating a predicted property score for the protein and the ligand using a property prediction neural network. For convenience, the process 200 will be described as being performed by a system of one or more computers located in one or more locations. For example, a property prediction system, e.g., the property prediction system 100 of FIG. 1, appropriately programmed in accordance with this specification, can perform the process 200.

The system generates a protein-ligand embedding using an embedding neural network (202). The embedding neural network processes protein data and ligand data to generate a protein - ligand embedding that represents the protein and the ligand. The protein-ligand embedding can be a 2D array that represents the protein and the ligand.

The system processes the protein-ligand embedding using a property prediction neural network to generate a property score (204). The property prediction neural network is configured to process the protein - ligand embedding to generate a property score.

The property prediction neural network can have any appropriate neural network architecture that enables the property prediction neural network to perform its described functions. In particular, the property prediction neural network can include any appropriate types of neural network layers (e.g., fully connected layers, convolutional layers, attention layers, etc.) in any appropriate number (e.g., 5 layers, 10 layers, or 20 layers) and connected in any appropriate configuration (e.g., as a directed graph of layers).

For instance, the property prediction neural network can have an architecture that receives (as input) the amino acid - amino acid and atom - atom embeddings from the diagonal of the 2D array of embeddings representing the protein-ligand complex. The property prediction neural network can include a layer that generates a respective atom embedding for each atom in each amino acid in the protein, e.g., by processing: (i) the amino acid - amino acid embedding for the amino acid that includes the atom, and (ii) atom-specific data such as the elemental type of the atom, data identifying whether the atom is included in the protein backbone or in a sidechain, and so forth. The property prediction neural network can then process the atom embeddings of the atoms in the protein and in the ligand using a sequence of self-attention layers to generate updated atom embeddings, aggregate the atom embeddings by a pooling layer (e.g., that performs max pooling or average pooling) to generate an aggregated atom embedding, and then process the aggregated atom embedding by one or more fully-connected layers to generate the predicted property score.

The property score can characterize any appropriate property of the ligand and the protein, e.g., the property score can characterize one or more of: a likelihood of occurrence of a binding event that includes the protein and the ligand; or a binding affinity of the protein and the ligand; or a likelihood that the ligand is an agonist for the protein; or a likelihood that the ligand is an antagonist for the protein; or a potency of the ligand when acting on the protein; or a inhibitory effect of the ligand when acting on the protein.

In some examples, the binding affinity score can define the predicted binding affinity of the protein and the ligand as measured by a specified type of binding affinity assay. The type of binding affinity assay can be, for example, a surface plasmon resonance (SPR) assay, or a isothermal titration calorimetry (ITC) assay, or a fluorescence polarization (FP) assay, or an enzyme-linked immunosorbent assay (ELISA), etc.

The property prediction neural network can be configured to receive a network input that includes both: (i) the protein-ligand embedding, and (ii) data identifying a type of binding affinity assay. The property prediction neural network can then generate a predicted binding affinity associated with the type of assay specified in the network input.

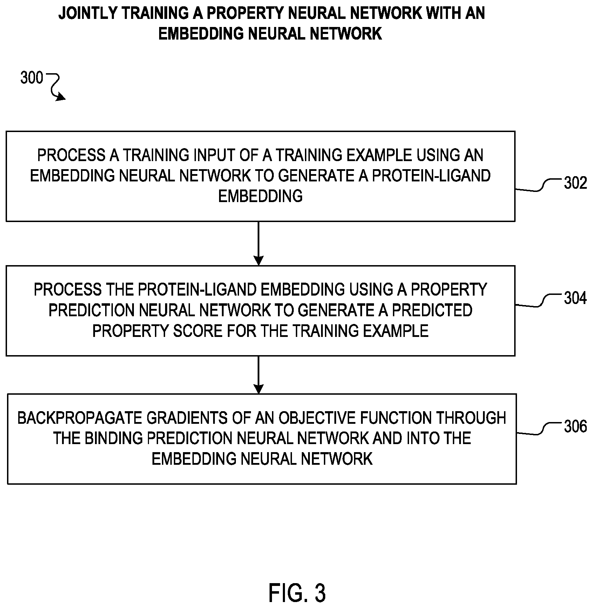

FIG. 3 is a flow diagram of an example process 300 for jointly training a property prediction neural network with the embedding neural network of the property prediction system. For convenience, the process 300 will be described as being performed by a system of one or more computers located in one or more locations. For example, a property prediction system, e.g., the property prediction system 100 of FIG. 1, appropriately programmed in accordance with this specification, can perform the process 300.

The property prediction neural network and the embedding neural network can be jointly trained on multiple training examples. Each training example corresponds to a respective protein - ligand complex and includes data defining: (i) a training input to the property prediction system, and (ii) a target output of the property prediction system. The training input to the property prediction system includes protein data (characterizing the protein of the protein - ligand complex) and ligand data (characterizing the ligand of the protein - ligand complex). The target output to the property prediction system can include a target property score.

For each training example, the system processes the training input of the training example using the embedding neural network to generate a protein-ligand embedding of the training protein and the training ligand (302).

The system processes the protein-ligand embedding of the training protein and the training ligand using the property prediction neural network to generate a predicted property score for the training protein and the training ligand of the training example (304).

The system backpropagates gradients of an objective function through the property prediction neural network and into the embedding neural network (306). The objective function can measure a discrepancy between: (i) the target property score specified by the training example, and (ii) the predicted property score generated by the embedding neural network and the property prediction neural network for the training example. The system can evaluate an objective function that measures an error (e.g., a root mean square deviation (RMSD), or a mean absolute error (MAE), or a mean squared error (MSE)) between: (i) the predicted property score, and (ii) the target binding affinity score.

The system can determine gradients of the objective function with respect to the parameters of the property prediction neural network and the embedding neural network using backpropagation. The system can then update the current values of the parameters of the embedding neural network and the property prediction neural network using the gradients, e.g., by the update rule of an appropriate gradient descent optimization algorithm, e.g., RMSprop or Adam.

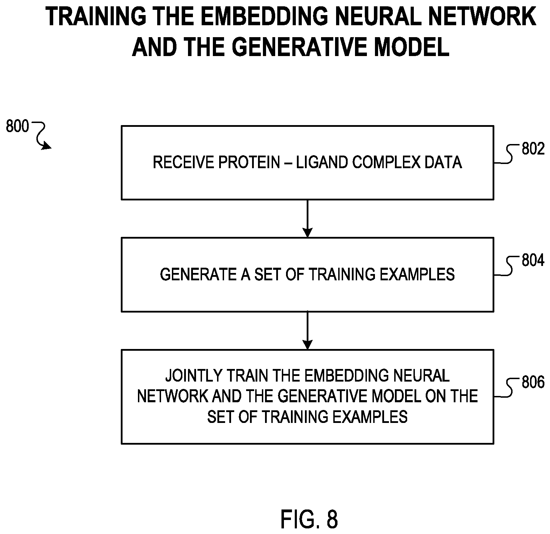

In addition to jointly training the embedding neural network jointly with the property prediction neural network, the system also trains the embedding neural network jointly with the generative model, e.g., as described with reference to FIG. 8. In some cases, the system can perform training over multiple stages including a first stage where the system jointly trains the embedding neural network and the generative model, and a second stage where the system jointly trains the embedding neural network and the property prediction neural network.

Training examples are available from publicly or commercially available databases, such as: ChEMBL(https://www.ebi.ac.uk/chembl/), which is a manually curated database of bioactive molecules with drug-like properties; BioLip, which is a ligand-protein binding database; DUD-E (https://dude.docking.org/); SitesBase; etc.

FIG. 4A is a flow diagram of an example process 400 for generating a predicted property score for a protein and a ligand using a graph neural network. For convenience, the process 400 will be described as being performed by a system of one or more computers located in one or more locations. For example, a property prediction system, e.g., the property prediction system 100 of FIG. 1, appropriately programmed in accordance with this specification, can perform the process 400.

The system generates a protein-ligand embedding using an embedding neural network (402). The embedding neural network processes protein data and ligand data to generate a protein - ligand embedding that represents the protein and the ligand. The protein-ligand embedding can be a 2D array that represents the protein and the ligand.

The system generates a predicted joint 3D structure of the protein and the ligand using a generative model and when the generative model is conditioned on the protein-ligand embedding (404). The predicted joint 3D structure of the protein and the ligand can define a respective predicted three-dimensional spatial location of each atom in the protein and of each atom in the ligand. An example process for generating a predicted 3D structure of a protein-ligand complex using a generative diffusion model is described with reference to FIG. 6.

The system generates data defining a graph representing at least a portion of the predicted joint 3D structure of the protein and the ligand (406). The graph includes a set of nodes and a set of edges, where each edge in the set of edges connects a respective pair of nodes in the set of nodes. An example process for generating a graph representing a 3D structure of a protein-ligand complex is described with reference to FIG. 4B.

The system processes the graph representing at least the portion of the predicted joint 3D structure of the protein and the ligand using a graph neural network to generate the property score that defines the predicted property of the protein and the ligand (408). The graph neural network is configured to process a graph that includes edges and nodes to generate a property score.

The graph neural network can have any appropriate neural network architecture that enables the graph neural network to perform its described functions. In particular, the graph neural network can include any appropriate types of neural network layers (e.g., message passing layers, fully connected layers, convolutional layers, attention layers, etc.) in any appropriate number (e.g., 5 layers, 10 layers, or 20 layers) and connected in any appropriate configuration (e.g., as a directed graph of layers).

In some implementations, the graph neural network includes multiple message passing layers. The message passing layers can allow neighboring edges and nodes to exchange information and influence each other e.g., by using an update function. For example, the graph neural network can include 3 message passing layers that are stacked together. Each node in the graph can eventually incorporate information from nodes that are 3 steps away from it due to stacking 3 message passing layers together.

An example process for processing the graph representing the 3D structure of the protein-ligand complex using the graph neural network to generate the predicted property score is described below with reference to FIG. 4C.

FIG. 4B is a flow diagram of an example process 410 for generating a graph based on a predicted 3D structure of a protein-ligand complex. For convenience, the process 410 will be described as being performed by a system of one or more computers located in one or more locations. For example, a property prediction system, e.g., the property prediction system 100 of FIG. 1, appropriately programmed in accordance with this specification, can perform the process 410.

The system receives data defining a predicted 3D structure of the protein-ligand complex (412). More specifically, the system receives a respective set of atom features for each atom in the complex. The set of atom features for an atom define the 3D spatial position of the atom in the protein-ligand complex, and optionally, define one or more additional features of the atom, e.g., including one or more of: an element type of the atom, a partial charge of the atom, or a hybridization state of the atom. The predicted 3D structure of the complex may be generated by a generative model that is conditioned on a protein-ligand embedding.

The system instantiates a set of atom nodes for inclusion in the graph (414). Each atom node in the set of atom nodes represents a respective atom in the protein or in the ligand. In particular, the system generates a respective atom node representing each atom in the ligand, and the system generates a respective atom node representing at some of the atoms in the protein.

In some implementations, the system generates a respective atom node representing each atom in the protein, i.e., so that every atom in the protein is represented by a respective atom node in the graph. In other implementations, the selects a proper subset of the atoms in the protein to be represented in the graph, e.g., rather than representing all the atoms in the protein in the graph. For instance, the system can identify a proper subset of the atoms in the protein as being included in the binding pocket to which the ligand is bound, and then generate a respective atom node in the graph for only those atoms in the protein that are included in the binding pocket. In particular, the system can refrain from generating atom nodes in the graph representing the atoms in the protein that are outside the binding pocket. The system can identify the binding pocket in the protein in any of variety of possible ways. For instance, the system can determine that any atom in the protein that is within a threshold distance (e.g., 2 Angstroms) of at least one atom in the ligand (i.e., in the 3D structure of the protein-ligand complex) is included in the binding pocket of the protein.

Generating a graph that represents only a proper of the atoms in the protein, e.g., only the atoms in the binding pocket of the protein, can reduce consumption of computational resources during processing of the graph by the graph neural network. Further, training the graph neural network to predicting protein-ligand properties based on graphs that represent only the binding pocket of the protein (i.e., as opposed to the whole protein) can improve the generalization performance of the graph neural network, e.g., by reducing the likelihood of the graph neural network overfitting the training data by learning to memorize irrelevant features of the protein that are distant from the binding pocket.

Optionally, the system generates a set of super nodes for inclusion in the graph. **In** contrast to atom nodes, which represent individual atoms in the complex (as described above), a super node represents a higher-level entity in the complex such as an amino acid or a structural motif.

More specifically, the system can generate a respective super node representing some or all of the amino acids in the protein. For instance, the system can generate respective super node for each amino acid in the entire protein, or the system can generate a respective super node for only those amino acids in the protein that are included in the binding pocket of the protein. (The system can determine that an amino acid is included in the binding pocket of the protein, e.g., if the amino acid includes at least one atom that is in the binding pocket of the protein).

Further, the system can generate a respective super node representing each structural motif in the ligand. More specifically, the system can partition the atoms in the ligand into multiple groups of atoms that each represent a respective structural motif from a set of possible structural motifs. A structural motif refers to a specific combination and arrangement of atoms in a molecule, and the set of possible structural motifs can include one or more of: aromatic rings, chelating groups, sulfonamides, and so forth. The system can generate a respective super node representing each structural motif in the ligand. Thus, for instance if the ligand includes 100 atoms that are partitioned into 10 structural motifs, the system can generate 10 super nodes, each of which represents a respective structural motif in the ligand.

The system instantiates a set of edges in the graph (416). For each pair of atom nodes in the graph, the system can determine that the pair of atom nodes should be connected by an edge in the graph if the corresponding pair of atoms represented by the pair of atom nodes are separated by less than a threshold distance (e.g., 2 Angstroms, or 5 Angstroms, or 10 Angstroms) in the 3D structure of the complex.

The system can instantiate edges that fully connect any super nodes in the graph, i.e., such that every pair of super nodes in the graph is connected by an edge in the graph. In particular, the system can instantiate edges that connect: (i) each super node representing an amino acid in the protein to each other super node representing an amino acid in the protein, (ii) each super node representing an amino acid to each super node representing a structural motif in the ligand, and (iii) each super node representing a structural motif in the ligand to each other super node representing a structural motif in the ligand.

For each atom node that represents a respective atom in the protein, the system can instantiate an edge that connects the atom to the super node representing the amino acid that includes the atom.

For each atom node that represents a respective atom in the ligand, the system can instantiate an edge that connects the atom to the super node representing the structural motif that includes the atom.

The super nodes in the graph facilitate long range propagation of information across the graph as the graph is being processed by the message passing layers of the graph neural network. More specifically, the graph can include a large number of atom nodes, and only those atom nodes that are in separated by less than a threshold spatial distance in the 3D structure of the complex are connected by an edge. Thus the operations of the message passing layers, if reliant only on propagating information along edges connecting the atom nodes, may be unable to propagate information between spatially distant atoms in the complex. The system can address this issue by the inclusion of super nodes in the graph because the super nodes can greatly reduce the maximum "path length" between atom nodes in the graph to, e.g., such that the maximum path length between nodes in the graph is three. (The "path length" between a pair of atom nodes can refer to the minimum number of edges in a path connecting the pair of atom nodes in the graph). The inclusion of super nodes can thus allow the graph neural network to rapidly propagate information between all the atom nodes in the graph, even between those atom nodes that represent spatially distant atoms in the complex.

The system associates a respective set of features with each node in the graph (418). For instance, for each atom node in the graph, the system can associate a set of features with the atom node including one or more of: a feature indicating whether the atom node is included in the protein or in the ligand; a feature defining a 3D spatial position of the atom in the protein-ligand complex; a feature defining an elemental type of the atom; a feature defining a partial charge of the atom; a feature defining a hybridization state of the atom; and so forth. As another example, for each super node in the graph, the system can associate a set of features with the super node including one or more of: a feature indicating whether the super node represents an amino acid in the protein or a structural motif in the ligand; for super nodes representing amino acids in the protein, a feature representing a type of the amino acid represented by the super node; for super nodes representing structural motifs in the ligand, a feature representing a type of the structural motif represented by the super node.

Optionally, the system can associate a set of features with each edge in the graph. For instance, the set of features associated with an edge between a pair of atom nodes can include a feature representing the spatial distance separating the pair of atoms represented by the pair of atom nodes in the 3D structure of the complex. As another example, the set of features associated with an edge can include a feature that classifies whether the edge connects: a pair of atom nodes; or a pair of super nodes; or an atom node to a super node.

FIG. 4C is a flow diagram of an example process 420 for processing a graph representing a 3D structure of a protein-ligand complex using a graph neural network to generate a predicted protein-ligand property score. For convenience, the process 420 will be described as being performed by a system of one or more computers located in one or more locations. For example, a property prediction system, e.g., the property prediction system 100 of FIG. 1, appropriately programmed in accordance with this specification, can perform the process 420.

The system receives the graph representing the 3D structure of the protein-ligand complex (422). The graph includes a set of nodes and a set of edges, where each edge in the set of edges connects a respective pair of nodes in the set of nodes. The graph can include atom notes representing atoms in the complex, and optionally, super nodes that each represent a respective amino acid in the protein or a respective structural motif in the ligand. Each node in the graph can be associated with a respective set of node features, and optionally, each edge in the graph can be associated with a respective set of edge features.

The system generates a respective embedding associated with each node in the graph using an encoder block of the graph neural network (424). More specifically, for each node in the graph, the encoder block processes the set of node features associated with the node to generate an embedding for the node. Optionally, for each edge in the graph, the encoder block can process the set of edge features associated with the edge to generate an embedding for the edge.

The system processes the node embeddings associated with the nodes in the graph by a sequence of one or more message passing neural network layers (426). Each message passing neural network layer is configured to receive a set of current node embeddings associated with the nodes in the graph, to process the set of input node embeddings in accordance with values of a set of message passing neural network layer parameters and using operations that are conditioned on the topology of the graph, and to generate a respective updated node embedding associated with each node in the graph. For instance, for each node in the graph, a message passing layer can generate an updated node embedding for the node based on: (i) the current node embedding for the node, and (ii) the current node embeddings of any neighboring nodes of the node. (A "neighboring" node of a given node refers to node that is directly connected to the given node by an edge in the graph).

The first message passing neural network layer can receive the node embeddings generated by the encoder block of the graph neural network layer, as described at step 424. Each subsequent message passing neural network layer can receive the node embeddings generated as an output by the preceding message passing neural network layer.

Optionally, each message passing neural network layer can update the edge embeddings associated with the edges in the graph. For instance, a message passing neural network layer can update the edge embedding associated with an edge in the graph based on: (i) the edge embedding associated with the edge, and (ii) the node embeddings of the nodes that are connected by the edge.

The system processes the updated node embeddings generated by the final message passing neural network layer using an output block of the graph neural network to generate the predicted property score (428). For instance, the output block can pool (e.g., average, sum, or otherwise combine) the updated node embeddings (and, optionally, the updated edge embeddings) to generate a combined embedding, and process the combined embedding by a sequence of fully connected layers to generate the predicted property score.

In some implementations, the graph neural network can be configured to receive a network input that includes both: (i) the graph representing the 3D structure of the protein-ligand complex, and (ii) data identifying a type of binding affinity assay. The graph neural network can then generate a predicted binding affinity associated with the type of assay specified in the network input. For instance, the output block of the graph neural network can jointly process the combined embedding and the data identifying the type of binding affinity assay to generate a predicted binding affinity associated with the type of assay specified in the network input.

FIG. 5 is a flow diagram of an example process 500 for training a graph neural network. For convenience, the process 500 will be described as being performed by a system of one or more computers located in one or more locations. For example, a property prediction system, e.g., the property prediction system 100 of FIG. 1, appropriately programmed in accordance with this specification, can perform the process 500.

The system receives a set of training examples (502). Each training example corresponds to a respective protein - ligand complex and includes data defining: (i) a training input to the graph neural network, and (ii) a target output of the graph neural network. The training input to the graph neural network includes graph data defining a graph representing at least a portion of the predicted joint 3D structure of the protein and the ligand. The target output of the graph neural network can include a target property score.

The system trains the graph neural network using the training examples to optimize an objective function (504). For each training example, the objective function can measure a discrepancy between: (i) the target property score specified by the training example, and (ii) the predicted property score generated by the graph neural network for the training example. The objective function can measure an error between: (i) the predicted property score, and (ii) the target property score. The objective function can be, e.g., a squared error objective function (e.g., when the target property score represents a continuous value such as a binding affinity) or a cross-entropy objective function (e.g., when the target property score represents a discrete value, e.g., for whether a binding event occurs, or a discrete value defining whether the ligand is an agonist or an antagonist for the protein).

FIG. 6 is a flow diagram of an example process 600 for generating a predicted joint 3D structure of a protein and a ligand and, optionally, a predicted property score of the protein and the ligand, using a generative diffusion model that includes a denoising neural network. For convenience, the process 600 will be described as being performed by a system of one or more computers located in one or more locations. For example, a property prediction system, e.g., the property prediction system 100 of FIG. 1, appropriately programmed in accordance with this specification, can perform the process 600.

For convenience, the process 600 will describe jointly generating the 3D structure of the protein-ligand complex and the predicted protein-ligand property score. In some implementations, the generative diffusion model generates only the predicted 3D structure of the protein-ligand complex without generating the predicted property score. In these implementations, the generative diffusion model excludes the property score from the iterative denoising process implemented using the denoising neural network.

The system generates a respective initial 3D spatial position for each atom in the complex comprising the protein and the ligand (602). For instance, for each atom in the complex, the system can sample a 3D spatial position of atom from a probability distribution over 3D space, e.g., a standard Normal distribution over 3D space.

The system generates property score data defining a respective initial property score of the protein and the ligand (604). For instance, the system can sample an initial property score from a probability distribution, e.g., a uniform distribution over a predefined range of values.

The system performs steps 606 - 608, which are described next, over a sequence of iterations that may be referred to as "time steps". The description of steps 606 - 608 (and the related steps 610 and 614) which follows will reference a "current" time step for convenience; the current time step can be any time step in the sequence of time steps. The system can perform the steps 606 - 608 over any appropriate number of time steps, e.g., 3 time steps, 10 time steps, or 100 time steps. The number of time steps can be a predetermined number of time steps.

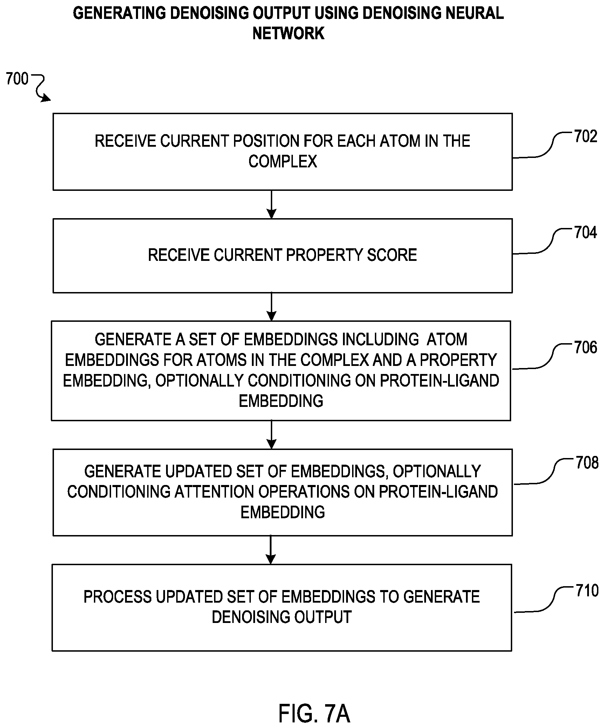

The system generates a denoising output using a denoising neural network that is conditioned on the protein - ligand embedding (606). The denoising output can be any appropriate data that enables estimation of the "final" 3D spatial position of each atom in the complex and of the "final" property score of the protein and the ligand. For instance, the denoising output can define, for each atom in the complex, a predicted error in the 3D spatial position of the atom at the current time step and a respective predicted error in the predicted property score of the protein and the ligand at the current time step. As another example, the denoising output can directly define a predicted property score of the protein and the ligand as well as, for each atom in the complex, a predicted 3D spatial position of the atom. As another example, the denoising output can define both, (i) a predicted error in the in the predicted property score of the protein and the ligand at the current time step, and (ii) a predicted property score of the protein and the ligand as well as, for each atom in the complex, both: (i) a predicted error in the 3D spatial position of the atom at the current time step, and (ii) a predicted 3D spatial position of the atom. As another example, the denoising output can define, a prediction for a value that is a linear combination of: (i) an actual property score of the protein and the ligand, and (ii) an error between the property score of the protein and the ligand at the current time step and the actual property score of the protein and the ligand as well as for each atom in the complex, a prediction for a value that is a linear combination of: (i) an actual 3D spatial position of the atom, and (ii) an error between the 3D spatial position of the atom at the current time step and the actual 3D spatial position of the atom, e.g., as implemented by the v-parametrization described in: Tim Salimans, Jonathan Ho, "Progressive distillation for fast sampling of diffusion models," ICLR 2022, arXiv:2202.00512v2. An example process for generating a denoising output using the denoising neural network is described in more detail with reference to FIG. 7A.

The system generates an initial estimate of the 3D spatial position for each atom in the complex and the property score of the protein and the ligand using at least the denoising output generated by the denoising neural network (608). The system can generate the initial estimate of the 3D spatial position for each atom in the complex and the property score of the protein and the ligand in any appropriate way, depending on the form of the denoising output. A few example techniques for generating the initial estimate of the 3D spatial position for each atom in the complex and the property score of the protein and the ligand using the denoising output are described next.

In one example, the denoising output defines, for each atom, a respective prediction for the 3D spatial position of the atom as well as a prediction of the property score of the protein and the ligand. In this example, the respective predicted 3D spatial position for each atom defines the initial estimate of the 3D spatial position for the atom and the predicted property score defines the initial estimate of the property score of the protein and the ligand.

In another example, the denoising output defines, for each atom, a predicted error in the 3D spatial position of the atom at the current time step as well as a predicted error in the property score of the protein and the ligand at the current time step. In this example, the system can generate the initial estimate for the 3D spatial position for each atom as a linear combination of: (i) the current 3D spatial position of the atom, and (ii) the predicted error in the 3D spatial position of the atom. Each term in the linear combination can be scaled by a respective constant value that is dependent on the time step. For instance, the system can generate the initial estimate for the 3D spatial position $x_{t-1}$ for an atom in the complex as:

$$x_{t-1} = \alpha_t^{-0.5}\left(x_t - (1 - \alpha_t)(1 - \bar{\alpha}_t)^{-0.5}\epsilon_\theta(x_t, t)\right) \qquad (1)$$

where $t$ indexes the current time step, $\alpha_t$, $\bar{\alpha}_t$, and $\sigma_t$ are constants specific to time step $t$, and $\varepsilon_\theta(x_t, t)$ is the predicted error in the 3D spatial position of the atom (e.g., as generated by the denoising neural network at the time step). (In the notation of equation (1), the time steps decrement, such that time step $t - 1$ is the "next" time step after time step $t$). The constants in equation (1) ($\alpha_t$, $\bar{\alpha}_t$, and $\sigma_t$) can be selected in accordance with a predefined noise schedule. Similarly, the system can generate the initial estimate for the property score of the protein as a linear combination of: (i) the current property score of the protein and the ligand, and (ii) the predicted error in the property score of the protein and the ligand. Each term in the linear combination can be scaled by a respective constant value that is dependent on the time step. For instance, the system can generate the initial estimate for the property score $b_{t-1}$ for an atom in the complex as:

$$b_{t-1} = \alpha_t^{-0.5}\left(b_t - (1 - \alpha_t)(1 - \bar{\alpha}_t)^{-0.5}\epsilon_\theta(b_t, t)\right) \qquad (2)$$

where $t$ indexes the current time step, $\alpha_t$, $\bar{\alpha}_t$, and $\sigma_t$ are constants specific to time step $t$, and $\varepsilon_\theta(b_t, t)$ is the predicted error in the property score of the protein and the ligand (e.g., as generated by the denoising neural network at the time step).

In another example, the denoising output defines, for each atom, both: (i) a predicted 3D spatial position of the atom, and (ii) a predicted error in the 3D spatial position of the atom at the current time step; as well as (i) a predicted property score of the protein and the ligand, and (ii) a predicted error in the property score of the protein and the ligand at the current time step. In this example, the system can generate the initial estimate for the 3D spatial position of the atom as a combination (e.g., an average) of: (i) the predicted 3D spatial position of the atom as specified by the denoising output, and (ii) a predicted 3D spatial position of the atom that is derived from the predicted error in the 3D spatial position of the atom at the current time step, e.g., using equation (1). In this example, the system can generate the initial estimate for the property score of the protein and the ligand as a combination (e.g., an average) of: (i) the property score of the protein and the ligand as specified by the denoising output, and (ii) a predicted property score of the protein and the ligand that is derived from the predicted error in the property score of the protein and the ligand at the current time step, e.g., using equation (2).

In another example, the denoising output is expressed using a v-parametrization, and the system generates a respective initial estimate for the 3D spatial position for each atom as well as the property score of the protein and the ligand using the techniques described in Tim Salimans, Jonathan Ho, "Progressive distillation for fast sampling of diffusion models," ICLR 2022, arXiv:2202.00512v2.

Optionally, the system can generate a respective confidence measure for the initial estimate of the respective 3D spatial position of each atom in the complex. For instance, as part of generating the denoising output, the denoising neural network can generate a respective atom embedding for each atom in the complex, e.g., as the output of the update block of the denoising neural network. The system can process each atom embedding using one or more neural network layers (e.g., a combination of one or more of: fully connected layers, or attention layers, or pooling layers) to generate a respective confidence estimate for the initial estimate of the 3D spatial position of the atom(s) represented by the atom embedding. The confidence measure for the initial estimate of the 3D spatial position of an atom can characterize a predicted error in the initial estimate of the 3D spatial position of the atom.

Optionally, the system can generate a respective confidence measure for the initial estimates of the 3D spatial positions of pairs of atoms in the complex. For instance, for a first atom in the complex and a second atom in the complex, the system can process an atom embedding representing the first atom and an atom embedding representing the second atom (e.g., as generated by the update block of the denoising neural network) using one or more neural network layers (e.g., a combination of one or more of: fully connected layers, or attention layers, or pooling layers) to generate a confidence estimate for the initial estimates of the 3D spatial positions of the first atom and the second atom. The confidence measure can characterize, e.g., a predicted error in the relative 3D displacement of the first atom and the second atom.

Optionally, the system can generate a confidence measure for the structure of the protein as defined by the initial estimates of the 3D spatial positions of the atoms in the protein, e.g., by combining (e.g., summing or averaging) the confidence measures for the individual atoms included in the protein.

Optionally, for each ligand, the system can generate a confidence measure for the structure of the ligand as defined by the initial estimates of the 3D spatial positions of the atoms in the ligand, e.g., by combining (e.g., summing or averaging) the confidence measures for the individual atoms included in the ligand.

Optionally, for each ligand, the system can generate a confidence measure for the structure of an interface between the protein and the ligand, e.g., by combining (e.g., summing or averaging) the confidence measures of pairs of atoms included in the interface. A pair of atoms can be referred to as being included in the interface, e.g., if the pair includes: (i) a first atom included in the ligand, and (ii) a second atom included in the protein, where the relative displacement between the 3D spatial positions of the atoms is less than a threshold, e.g., 2 Angstroms, or 3 Angstroms, or 8 Angstroms. An example of generating a confidence measure for a pair of atoms is described above.

Optionally, the system can generate a respective confidence measure for the initial estimate of the property score of the protein and the ligand. For instance, as part of generating the denoising output, the denoising neural network can generate a property score embedding, e.g., as the output of the update block of the denoising neural network. The system can process the property score embedding using one or more neural network layers (e.g., a combination of one or more of: fully connected layers, or attention layers, or pooling layers) to generate a confidence estimate for the initial estimate of the property score of the protein and the ligand represented by the property score embedding. The confidence measure for the initial estimate of property score of the protein and the ligand can characterize a predicted error in the initial estimate of the property score of the protein and the ligand.

If the current time step is the final time step (i.e., in the sequence of denoising time steps), the system outputs the initial estimate of the 3D spatial position of each atom in the complex as the predicted joint 3D structure of the complex and the initial estimate of the property score of the protein and the ligand as the predicted property score of the protein and the ligand (612).

If the current time step is not the final time step, the system generates a respective 3D spatial position for each atom for the next time step based on the initial estimates of the 3D spatial positions of the atoms (as generated at step 608) and a property score for the next time step based on the initial estimate of the property score (as generated at step 608) using an appropriate diffusion sampling technique (614). A few examples of possible diffusion sampling techniques are described next.

In one example, the system can generate the 3D spatial position of each atom in the complex at the next time step by combining random noise with the initial estimate of the 3D spatial position of the atom. For instance, for each atom, the system can add respective random noise to the initial estimate of the 3D spatial position of the atom. The random noise can be sampled from a probability distribution over 3D space. The probability distribution over 3D space can vary based on the time step, e.g., such that the variance of the noise combined with the updated 3D spatial positions of the atoms decreases over the sequence of time steps. In this, example the system can generate the predicted property score of the protein and the ligand at the next time step by combining random noise with the initial estimate of the property score of the protein and the ligand. The random noise can be sampled from a probability distribution.

As another example, the system generate the 3D spatial position of each atom in the complex at the next time step and the property score at the next time step using a deterministic diffusion sampling technique, i.e., that does not rely on random noise. An example of a deterministic diffusion sampling technique is the denoising diffusion implicit model (DDIM), e.g., as described in: Jiaming Song, Chenlin Meng, Stefano Ermon, "Denoising diffusion implicit models," ICLR 2021, arXiv:2010.02502v4.

Optionally, the system can perform the process 600 multiple times to generate multiple predictions for the joint 3D structure of the complex and the property score of the protein and the ligand. Each execution of the steps of the process 600 can result in the generation of a different predicted joint 3D structure of the complex and a different property score of the protein and the ligand, e.g., as a result of stochasticity in the random sampling performed to generate the initial positions of the atoms (at step 602) and the initial property score (at step 604), and in some cases, as a result of stochasticity in diffusion sampler (at step 614).

FIG. 7A is a flow diagram of an example process 700 for generating a denoising output using a denoising neural network. For convenience, the process 700 will be described as being performed by a system of one or more computers located in one or more locations. For example, a property prediction system, e.g., the property prediction system 100 of FIG. 1, appropriately programmed in accordance with this specification, can perform the process 700.

The system receives: (i) data defining a respective current 3D spatial position of each atom in the complex, and (ii) the protein - ligand embedding (702). The protein - ligand embedding can include: (i) atom - atom embeddings, (ii) amino acid - amino acid embeddings, and (iii) amino acid - atom embeddings. The system can optionally receive additional inputs, e.g., an input defining a current time step in a diffusion process being implemented by a generative diffusion model.

The system receives current property score data that defines a current predicted property score of the protein and the ligand (704). The current property score of the protein and the ligand can be, for example, a binding affinity score.

The system generates a respective atom embedding for each atom in the complex and a property score embedding using an encoder block of the generative neural network (706).

The respective atom embeddings are based at least in part on the current 3D spatial position of the atom. Optionally, the system can generate the atom embeddings for the atoms based at least in part on the protein - ligand embedding (and, optionally, the current time step in the diffusion process), e.g., in addition to the current 3D spatial positions of the atoms. For instance, for each atom, the system can generate the atom embedding of the atom based on both: (i) the current 3D spatial position of the atom, and (ii) a respective conditioning embedding selected from the collection of embeddings included in the protein - ligand embedding. For an atom included in an amino acid in the complex, the conditioning embedding for the atom can be the amino acid - amino acid embedding (i.e., from the protein - ligand embedding) of the amino acid that includes the atom (i.e., the amino acid - amino acid embedding corresponding to a pair of amino acids that includes two copies of the amino acid). For an atom included in a ligand in the complex, the conditioning embedding for the atom can be the atom - atom embedding (i.e., from the protein - ligand embedding) of the atom (i.e., the atom - atom embedding corresponding to a pair of atoms that includes two copies of the atom).

The system can generate the atom embedding for an atom based on the current 3D spatial position of the atom (and, optionally, a conditioning embedding for the atom) in any of a variety of possible ways. For instance, the system can generate the atom embedding for an atom by processing the 3D spatial position of the atom using an encoder block of the denoising neural network. As another example, the system can generate the atom embedding for an atom by processing both: (i) the 3D spatial position of the atom, and (ii) the conditioning embedding for the atom, using an encoder subnetwork of the denoising neural network. As another example, the system can generate the atom embedding for an atom by concatenating: (i) an output generated by an encoder subnetwork of the denoising neural network by processing the 3D spatial position of the atom, and (ii) the conditioning embedding for the atom.

The property score embedding is based at least in part on the current predicted property score of the protein and the ligand at the time step. Optionally, when the property score is a binding affinity score, the system can generate the property score embedding based at least in part on data identifying a type of binding affinity assay (and, optionally, the current time step in the diffusion process), e.g., in addition to the current binding affinity of the protein and the ligand. For instance, the system can generate the binding affinity embedding based on both: (i) the current binding affinity of the protein and the ligand, and (ii) data identifying a type of binding affinity assay.

The system can generate the binding affinity embedding based on the current property score of the protein and the ligand (and, optionally, a data identifying a type of binding affinity assay) in any of a variety of possible ways. For instance, the system can generate the property score embedding by processing the property score of the protein and the ligand using an encoder block of the denoising neural network. As another example, the system can generate the binding affinity embedding for an atom by processing both: (i) the binding affinity of the protein and the ligand, and (ii) the data identifying the type of binding affinity assay, using an encoder subnetwork of the denoising neural network. As another example, the system can generate the binding affinity embedding by concatenating: (i) an output generated by an encoder subnetwork of the denoising neural network by processing the binding affinity of the protein and the ligand, and (ii) data identifying the type of binding affinity assay.

The encoder block of the denoising neural network can include any appropriate types of neural network layers (e.g., fully connected layers, convolutional layers, and so forth) in any appropriate number (e.g., 1 layer, 3, layers or 5 layers) and connected in any appropriate configuration (e.g., as a directed graph of layers). In a particular example, the encoder block includes a sequence of fully connected neural network layers and is configured to, for each atom, process data defining the 3D spatial position of the atom and the conditioning embedding for the atom to generate the atom embedding of the atom as well as process data defining the binding affinity of the protein and the ligand and the data identifying the type of binding affinity assay to generate the binding affinity embedding.

In some implementations, for each amino acid in the protein, the system can generate one atom embedding that jointly represents all the atoms in the amino acid. Thus, in these implementations, the number of atom embeddings may be equal to a sum of: (i) the number of atoms in the one or more ligands, and (ii) the number of amino acids in the protein. The system can generate an atom embedding that jointly represents all the atoms in an amino acid in any appropriate way. For instance, the system can generate a respective atom embedding for each atom in the amino acid (as described above), and then combine the atom embeddings for the atoms in the amino acid, e.g., using a pooling operation (e.g., a max pooling or a summation pooling operation), or by processing the atom embeddings for the atoms in the amino acid using one or more neural network layers (e.g., fully connected layers or self-attention layers) to generate the embedding that jointly represents all the atoms in the amino acid. Generating atom embeddings that jointly represent all the atoms in an amino acid can significantly reduce the overall number of atom embeddings and thus reduce consumption of computational resources, e.g., memory and computing power, resulting from operations performed by an update block of the denoising neural network, as will be described next.

The system processes the atom embeddings for the atoms in the complex and the property score embedding, using an update block of the denoising neural network, to generate a respective updated atom embedding for each atom in the complex and an updated property score embedding (708). The update block of the denoising neural network layer can include a sequence of self-attention blocks. Each self-attention block can be configured to receive a respective current atom embedding for each atom in the complex and a current property score embedding, to apply a self-attention operation to the current atom embeddings of the atoms in the complex and current property score embedding, and to provide the updated atom embeddings and updated property score embedding, e.g., for processing by a subsequent neural network layer.

In some implementations, each self-attention block updates the property score embedding using attention over: (i) the property score embedding, and (ii) all the atom embeddings.

In some implementations, each self-attention block updates each atom embedding over all the atom embeddings but not over the property score embedding.

In some implementations, each self-attention block updates each atom embedding over: (i) all the atom embeddings, and (ii) the property score embedding.

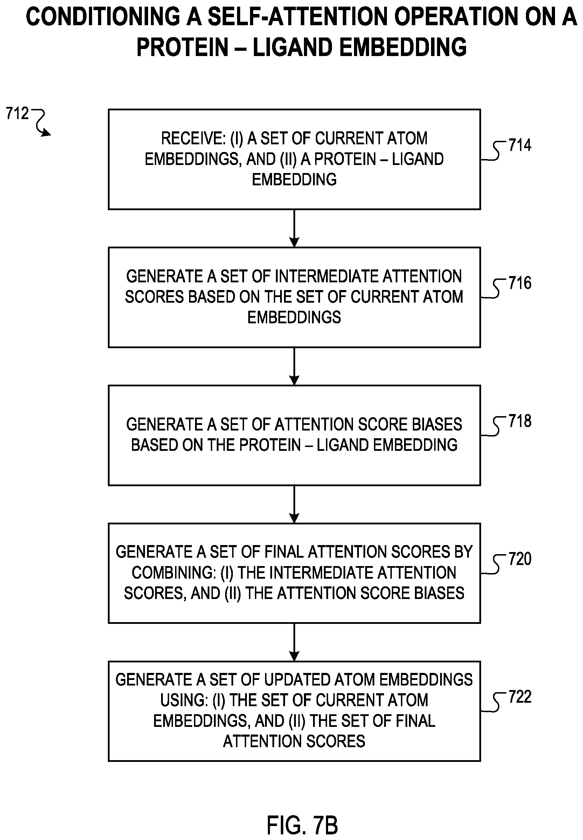

Each self-attention block included in the update block can apply any appropriate self-attention operation to the current atom embeddings of the atoms in the complex and the current property score embedding, e.g., a single-head or multi-head query-key-value (QKV) self-attention operation. Optionally, the system can condition the self-attention operations of one or more of the self-attention blocks on the protein - ligand embedding. An example process for implementing a self-attention operation over the set of atom embeddings that is conditioned on the protein - ligand embedding is described in more detail with reference to FIG. 7B.

The update block of the denoising neural network can include any appropriate number of self-attention blocks (e.g., 1 self-attention block, or 10 self-attention blocks, or 50 self-attention blocks) and can optionally include additional neural network layers of any appropriate type (e.g., fully connected layers, convolutional layers, and so forth) in any appropriate number (e.g., 1 layer, 3, layers or 5 layers) and connected in any appropriate configuration (e.g., interleaved with the self-attention blocks).

The system processes the updated atom embeddings and updated property score embedding (i.e., as generated by the update block of the denoising neural network) using a decoder block of the denoising neural network to generate the denoising output (710). Examples of denoising outputs are described above with reference to FIG. 6. The decoder block of the denoising neural network can include any appropriate types of neural network layers (e.g., fully connected layers, convolutional layers, and so forth) in any appropriate number (e.g., 1 layer, 3, layers or 5 layers) and connected in any appropriate configuration (e.g., as a directed graph of layers).

In a particular example, the decoder block can include a sequence of fully connected neural network layers that are configured to operate separately on each updated atom embedding to generate a predicted error in the 3D spatial position of the corresponding atom at the current time step, or to generate a predicted 3D spatial position of the corresponding atom, or both. The decoder block can further include a sequence of fully connected neural network layers that are configured to process the update property score embedding to generate, e.g., a predicted error in the current predicted property score at the current time step, or to generate a predicted property score, or both.

In implementations where one atom embedding jointly represents all the atoms in an amino acid (as described above with reference to step 706), the decoder block can process that (updated) atom embedding to generate respective denoising outputs for all the atoms in the amino acid. For instance, the decoder block can process an updated atom embedding that jointly represents all the atoms in an amino acid to generate a respective predicted error in the 3D spatial position of each atom in the amino acid, or to generate a respective 3D spatial position of each atom in the amino acid, or both.

FIG. 7B is a flow diagram of an example process 712 for updating a set of current atom embeddings using a self-attention operation that is implemented by a self-attention block of the denoising neural network and that is conditioned on a protein - ligand embedding. For convenience, the process 712 will be described as being performed by a system of one or more computers located in one or more locations. For example, a property prediction system, e.g., the property prediction system 100 of FIG. 1, appropriately programmed in accordance with this specification, can perform the process 712.

The system receives: (i) a set of current atom embeddings, and (ii) a protein - ligand embedding (714). The set of current atom embeddings includes a respective atom embedding for each atom in the complex. (In some cases, for each amino acid in the protein, all the atoms in the amino acid are jointly represented by one atom embeddings). The set of current atom embeddings can be generated, e.g., by the encoder block of the denoising neural network or by a previous self-attention layer in the update block of the denoising neural network. The protein - ligand embedding can be generated by an embedding neural network. The protein - ligand embedding can include: (i) atom - atom embeddings, (ii) amino acid - amino acid embeddings, and (iii) amino acid - atom embeddings.

The system generates a set of intermediate attention scores based on the set of current atom embeddings (716). The set of intermediate attention scores includes a respective attention score for each pair of current atom embeddings from the set of current atom embeddings.

The system can generate the intermediate attention scores in any of a variety of possible ways. For instance, the system can generate a respective query embedding for each current atom embedding by processing the current atom embedding using a query neural network, e.g., as:

$$Q = W^Q \cdot E \qquad (3)$$

where $Q$ is a matrix where each column (or row) defines a respective query embedding, $W^Q$ is a matrix of parameter values (defining the query neural network in this example), and $E$ is a matrix where each column (or row) defines a respective current atom embedding. Further, the system can generate a respective key embedding for each current atom embedding by processing the current atom embedding using a key neural network, e.g., as:

$$K = W^K \cdot E \qquad (4)$$

where $K$ is a matrix where each column (or row) defines a respective key embedding, $W^K$ is a matrix of parameter values (defining the key neural network in this example), and $E$ is a matrix where each column (or row) defines a respective current atom embedding. The system can generate the intermediate attention scores based on the query embeddings and the key embeddings, e.g., as:

$$A = Q \cdot K^T \qquad (5)$$

where $A$ is a matrix of intermediate attention scores, $Q$ is the matrix of query embeddings, and $K$ is the matrix of key embeddings.

The system generates a set of attention score biases based on the protein - ligand embedding (718). The set of attention score biases includes a respective attention score bias for each pair of current atom embeddings from the set of current atom embeddings.

The system can generate the set of attention score biases in any of a variety of possible ways. For instance, for each pair of current atom embeddings, the system can generate the attention score bias for the pair of current atoms embeddings by processing a corresponding conditioning embedding selected from the collection of embeddings included in the protein - ligand embedding using a projection neural network.

For a pair of current atom embeddings that includes: (i) a first current atom embedding representing a first atom included in a ligand, and (ii) a second current atom embedding representing a second atom included in a ligand, the conditioning embedding can be the atom - atom embedding for the first atom and the second atom in the protein - ligand embedding.

For a pair of current atom embeddings that includes: (i) a first current atom embedding representing an atom included in an amino acid, and (ii) a second current atom embedding representing an atom included in a ligand, the conditioning embedding can be the amino acid - atom embedding corresponding to: (i) the amino acid that includes the first atom, and (ii) the second atom, in the protein - ligand embedding.

For a pair of current atom embeddings that includes: (i) a first current atom embedding representing an atom included in a first amino acid, and (ii) a second current atom embedding representing an atom included in a second amino acid, the conditioning embedding can be the amino acid - amino acid embedding corresponding to: (i) the first amino acid, and (ii) the second amino acid, in the protein - ligand embedding.

For a pair of current atom embeddings that includes: (i) a first current atom embedding that jointly represents the atoms in a first amino acid, and (ii) a second current atom embedding that jointly represents the atoms in a second amino acid, the conditioning embedding can be the amino acid - amino acid embedding corresponding to: (i) the first amino acid, and (ii) the second amino acid, in the protein - ligand embedding.

For a pair of current atom embeddings that includes: (i) a first current atom embedding that jointly represents the atoms in an amino acid, and (ii) a second current atom embedding that represents an atom included in a ligand, the conditioning embedding can be the amino acid - atom embedding corresponding to: (i) the amino acid, and (ii) the atom, in the protein - ligand embedding.

The projection neural network can have any appropriate neural network architecture that enables the projection neural network to perform its described functions, e.g., processing a conditioning vector to generate an attention score bias. In particular, the projection neural network can include any appropriate number of neural network layers (e.g., 1 layer, or 5 layers, or 10 layers) in any appropriate number (e.g., 1 layer, 3, layers or 5 layers) and connected in any appropriate configuration (e.g., as a directed graph of layers).

The system generates a set of final attention scores by combining: (i) the intermediate attention scores, and (ii) the attention score biases (720). The set of final attention scores includes a respective final attention score for each pair of current atom embeddings in the set of current atom embeddings. The system can generate the final attention score for a pair of current atom embeddings by combining (e.g., summing): (i) the intermediate attention score for the pair of current atom embeddings, and (ii) the attention score bias for the pair of current atom embeddings. Optionally, the system can apply further processing operations to the set of final attention scores, e.g., by applying a soft-max operation to some or all of the final attention scores.

The system generates a set of updated atom embeddings using: (i) the set of current atom embeddings, and (ii) the set of final attention scores (722). For instance, to generate the set of updated atom embeddings, the system can generate a respective value embedding for each current atom embedding in the set of current atom embeddings by processing the current atom embedding using a value neural network, e.g., as:

$$V = W^V \cdot E \qquad (6)$$

where $V$ is a matrix where each column (or row) defines a respective value embedding, $W^V$ is a matrix of parameter values (defining the value neural network in this example), and $E$ is a matrix where each column (or row) defines a respective current atom embedding. The system can then generate the set of updated atom embeddings, e.g., as:

$$E' = V \cdot A \qquad (7)$$

where each column (or row) of $E'$ defines a respective updated atom embedding, each column (or row) of $V$ defines a respective value embedding, and $A$ denotes the set of final attention scores arranged into a matrix.

In implementations where the self-attention block implements a multi-head attention operation, each head of the attention operation can individually perform the steps of the process 600, and the updated atom embeddings generated by each attention head can be combined (e.g., concatenated) to define the overall output of the multi-head attention operation. Each attention head can have a respective set of neural network parameters, having values that are specific to each attention head, that are used for generating the intermediate attention scores and the attention score biases.

FIG. 8 is a flow diagram of an example process 800 for jointly training the embedding neural network and the generative model of the property prediction system. In the example process 800, the generative model is a model that implements a differentiable generative process. For instance, the generative model can be a generative diffusion model implemented using a denoising neural network, as described above with reference to FIG. 6. For convenience, the process 800 will be described as being performed by a system of one or more computers located in one or more locations. For example, a property prediction system, e.g., the property prediction system 100 of FIG. 1, appropriately programmed in accordance with this specification, can perform the process 800.

The system receives data characterizing a set of protein - ligand complexes (802). Each protein - ligand complex defines a joint 3D structure of a protein and one or more ligands, e.g., where each of the one or more ligands is bound to a respective binding site on the protein. Optionally, for each protein-ligand complex, the system receives data defining a property score of the protein and the ligand.

The system generates a set of training examples (804). Each training example corresponds to a respective protein - ligand complex and includes data defining: (i) a training input to the property prediction system, and (ii) a target output of the property prediction system. The training input to the property prediction system includes protein data (characterizing the protein of the protein - ligand complex) and ligand data (characterizing the one or more ligands of the protein - ligand complex). The target output of the property prediction system can be based on the joint 3D structure of the protein - ligand complex and the property score of the protein and the ligand.

The system jointly trains the embedding neural network and the generative model on the set of training examples by a machine learning training technique (806). More specifically, for each training example, the system can process the training input of the training example using the embedding neural network and the generative model to generate a predicted output of the generative model. The system can evaluate an objective function that measures an error (e.g., a root mean square deviation (RMSD), or a mean absolute error (MAE), or a mean squared error (MSE)) between: (i) the predicted output of the generative model, and (ii) the target output of the generative model. The system can determine gradients of the objective function with respect to the parameters of the embedding neural network and the generative model, e.g., using backpropagation. (The parameters of the generative model can include, e.g., a set of neural network parameters of a neural network implemented by the generative model). The system can then update the current values of the parameters of the embedding neural network and the generative model using the gradients, e.g., by the update rule of an appropriate gradient descent optimization algorithm, e.g., RMSprop or Adam.

Specific aspects of the training (e.g., the operations of the generative model during the training and the objective function) may depend on the implementation of the generative model. An example process for training a generative diffusion model that includes a denoising neural network on a training example is described in more detail next with reference to FIG. 9. In the example process of FIG. 9, the operations of the generative diffusion model are modified during training (e.g., as compared to the operations of the generative diffusion model during inference, e.g., as described with reference to FIG. 6), as will be described in more detail below.

In some implementations, the generative model includes one or more "confidence estimation" neural network layers that process atom embeddings generated by the generative model to generate confidence measures for the estimated positions of atoms or pairs of atoms in a complex. The system can jointly train the confidence estimation neural network layers along with the embedding neural network and the generative model. For instance, for each atom in the complex, the system can generate a confidence measure for the atom that defines a predicted error in the 3D spatial position of the atom, and the objective function can include a term that measures a difference between: (i) the predicted error in the 3D spatial position of the atom, and (ii) the actual error in the 3D spatial position of the atom. As another example, for each pair of atoms in the complex, the system can generate a confidence measure that defines a predicted error in the relative 3D displacement of the pair of atoms, and the objective function can include a term that measures a difference between: (i) the predicted relative 3D displacement of the pair of atoms, and (ii) the actual relative 3D displacement of the pair of atoms. The system can backpropagate gradients through the confidence estimation neural network layers, and optionally, into the generative model and/or the embedding neural network.

In some implementations, the generative model includes one or more confidence estimation neural network layers that process a property score embedding generated by the generative model to generate a confidence measure for the property score. The system can jointly train the confidence estimation neural network layers along with the embedding neural network and the generative model. For instance, the system can generate a confidence measure for the property score that defines a predicted error in the predicted property score, and the objective function can include a term that measures a difference between: (i) the predicted error in the property score, and (ii) the actual error in the property score.

FIG. 9 is a flow diagram of an example process 900 for jointly training an embedding neural network and a generative diffusion model on a training example. For convenience, the process 900 will be described as being performed by a system of one or more computers located in one or more locations. For example, a property prediction system, e.g., the property prediction system 100 of FIG. 1, appropriately programmed in accordance with this specification, can perform the process 900.

The system generates a protein - ligand embedding of the protein - ligand complex corresponding to the training example using the embedding neural network (902).

The system samples a time step from the sequence of denoising time steps (904). More specifically, during inference, the generative diffusion model can be configured to perform a sequence of denoising time steps, e.g., as described with reference to steps 606-608 of FIG. 6. During training, the system can randomly sample a single denoising time step from the sequence of denoising time steps, e.g., in accordance with a uniform distribution over the sequence of denoising time steps.

The system generates a respective noisy spatial position for each atom in the complex by combining random noise with the target spatial position of the atom (i.e., in the target 3D structure of the complex) and a noisy property score of the protein and the ligand (906). For instance, for each atom in the complex, the system can generate the noisy spatial position for the atom by adding random noise to the target spatial position of the atom. Similarly, the system can generate the noisy property score by adding random noise to the target property score for the protein and the ligand. The system can scale the random noise combined with the target spatial positions of the atoms by a constant that depends on the sampled time step, e.g., where the values of the constants corresponding to the denoising time steps are defined by a noise schedule. Similarly, the system can scale the random noise combined with the target property score for the protein and the ligand by a constant that depends on the sampled time step, e.g., where the values of the constants corresponding to the denoising time steps are defined by a noise schedule.

The system generates a denoising output using the denoising neural network while the denoising neural network is conditioned on the protein - ligand embedding (908). An example process for generating a denoising output is described in detail with reference to FIG. 7A. At step 702 of FIG. 7, the current position for each atom in the complex can be defined as the noisy spatial position for each atom in the complex. At step 704 of FIG. 7, the current property score of the protein and the ligand can be defined as the noisy property score for the protein and the ligand.

The system determines gradients of an objective function that depends on the denoising output, and uses the gradients to update the parameter values of the denoising neural network and the embedding neural network (910). The objective function can measure an error between: (i) the denoising output of the denoising neural network, and (ii) a target output of the denoising neural network. The target output of the denoising neural network can define an output of the denoising neural network that, if used to generate an initial estimate of the 3D spatial positions of the atoms in the complex (as described in step 608 of FIG. 6), would cause the initial estimate of the 3D spatial positions of the atoms to match the target (actual) 3D spatial positions of the atoms in the protein - ligand complex of the training example, and if used to generate an initial estimate of the property score of the protein and the ligand (as described in step 608 of FIG. 6), would cause the initial estimate of the property score of the protein and the ligand to match the target (actual) property score of the protein and the ligand of the training example.

This specification uses the term "configured" in connection with systems and computer program components. For a system of one or more computers to be configured to perform particular operations or actions means that the system has installed on it software, firmware, hardware, or a combination of them that in operation cause the system to perform the operations or actions. For one or more computer programs to be configured to perform particular operations or actions means that the one or more programs include instructions that, when executed by data processing apparatus, cause the apparatus to perform the operations or actions.

Embodiments of the subject matter and the functional operations described in this specification can be implemented in digital electronic circuitry, in tangibly-embodied computer software or firmware, in computer hardware, including the structures disclosed in this specification and their structural equivalents, or in combinations of one or more of them. Embodiments of the subject matter described in this specification can be implemented as one or more computer programs, i.e., one or more modules of computer program instructions encoded on a tangible non-transitory storage medium for execution by, or to control the operation of, data processing apparatus. The computer storage medium can be a machine-readable storage device, a machine-readable storage substrate, a random or serial access memory device, or a combination of one or more of them. Alternatively or in addition, the program instructions can be encoded on an artificially-generated propagated signal, e.g., a machine-generated electrical, optical, or electromagnetic signal, that is generated to encode information for transmission to suitable receiver apparatus for execution by a data processing apparatus.

The term "data processing apparatus" refers to data processing hardware and encompasses all kinds of apparatus, devices, and machines for processing data, including by way of example a programmable processor, a computer, or multiple processors or computers. The apparatus can also be, or further include, special purpose logic circuitry, e.g., an FPGA (field programmable gate array) or an ASIC (application-specific integrated circuit). The apparatus can optionally include, in addition to hardware, code that creates an execution environment for computer programs, e.g., code that constitutes processor firmware, a protocol stack, a database management system, an operating system, or a combination of one or more of them.

A computer program, which may also be referred to or described as a program, software, a software application, an app, a module, a software module, a script, or code, can be written in any form of programming language, including compiled or interpreted languages, or declarative or procedural languages; and it can be deployed in any form, including as a stand-alone program or as a module, component, subroutine, or other unit suitable for use in a computing environment. A program may, but need not, correspond to a file in a file system. A program can be stored in a portion of a file that holds other programs or data, e.g., one or more scripts stored in a markup language document, in a single file dedicated to the program in question, or in multiple coordinated files, e.g., files that store one or more modules, sub-programs, or portions of code. A computer program can be deployed to be executed on one computer or on multiple computers that are located at one site or distributed across multiple sites and interconnected by a data communication network.

In this specification the term "engine" is used broadly to refer to a software-based system, subsystem, or process that is programmed to perform one or more specific functions. Generally, an engine will be implemented as one or more software modules or components, installed on one or more computers in one or more locations. In some cases, one or more computers will be dedicated to a particular engine; in other cases, multiple engines can be installed and running on the same computer or computers.

The processes and logic flows described in this specification can be performed by one or more programmable computers executing one or more computer programs to perform functions by operating on input data and generating output. The processes and logic flows can also be performed by special purpose logic circuitry, e.g., an FPGA or an ASIC, or by a combination of special purpose logic circuitry and one or more programmed computers.

Computers suitable for the execution of a computer program can be based on general or special purpose microprocessors or both, or any other kind of central processing unit. Generally, a central processing unit will receive instructions and data from a read-only memory or a random access memory or both. The essential elements of a computer are a central processing unit for performing or executing instructions and one or more memory devices for storing instructions and data. The central processing unit and the memory can be supplemented by, or incorporated in, special purpose logic circuitry. Generally, a computer will also include, or be operatively coupled to receive data from or transfer data to, or both, one or more mass storage devices for storing data, e.g., magnetic, magneto-optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device, e.g., a universal serial bus (USB) flash drive, to name just a few.

Computer-readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks.

To provide for interaction with a user, embodiments of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's device in response to requests received from the web browser. Also, a computer can interact with a user by sending text messages or other forms of message to a personal device, e.g., a smartphone that is running a messaging application, and receiving responsive messages from the user in return.

Data processing apparatus for implementing machine learning models can also include, for example, special-purpose hardware accelerator units for processing common and compute-intensive parts of machine learning training or production, i.e., inference, workloads.

Machine learning models can be implemented and deployed using a machine learning framework, e.g., a TensorFlow framework, or a Jax framework.

Embodiments of the subject matter described in this specification can be implemented in a computing system that includes a back-end component, e.g., as a data server, or that includes a middleware component, e.g., an application server, or that includes a front-end component, e.g., a client computer having a graphical user interface, a web browser, or an app through which a user can interact with an implementation of the subject matter described in this specification, or any combination of one or more such back-end, middleware, or front-end components. The components of the system can be interconnected by any form or medium of digital data communication, e.g., a communication network. Examples of communication networks include a local area network (LAN) and a wide area network (WAN), e.g., the Internet.

The computing system can include clients and servers. A client and server are generally remote from each other and typically interact through a communication network. The relationship of client and server arises by virtue of computer programs running on the respective computers and having a client-server relationship to each other. In some embodiments, a server transmits data, e.g., an HTML page, to a user device, e.g., for purposes of displaying data to and receiving user input from a user interacting with the device, which acts as a client. Data generated at the user device, e.g., a result of the user interaction, can be received at the server from the device.

While this specification contains many specific implementation details, these should not be construed as limitations on the scope of any invention or on the scope of what may be claimed, but rather as descriptions of features that may be specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially be claimed as such, one or more features from a claimed combination can in some cases be excised from the combination, and the claimed combination may be directed to a subcombination or variation of a subcombination.

Similarly, while operations are depicted in the drawings and recited in the claims in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system modules and components in the embodiments described above should not be understood as requiring such separation in all embodiments, and it should be understood that the described program components and systems can generally be integrated together in a single software product or packaged into multiple software products.

Particular embodiments of the subject matter have been described. Other embodiments are within the scope of the following claims. For example, the actions recited in the claims can be performed in a different order and still achieve desirable results. As one example, the processes depicted in the accompanying figures do not necessarily require the particular order shown, or sequential order, to achieve desirable results. In some cases, multitasking and parallel processing may be advantageous.

Innovative aspects of the present disclosure are also set out in the following numbered clauses, which are not claims.

1. A method performed by one or more computers, the method comprising:

obtaining a network input that characterizes a protein and a ligand;
processing the network input characterizing the protein and the ligand using an embedding neural network to generate a protein-ligand embedding representing the protein and the ligand,
wherein the embedding neural network has been jointly trained with a generative model that is configured to:

receive an input protein-ligand embedding; and
generate, while conditioned on the input protein-ligand embedding, a predicted joint three-dimensional (3D) structure of an input protein and an input ligand represented by the input protein-ligand embedding; and

generating a property score that defines a predicted property of the protein and the ligand using the protein-ligand embedding.

2. The method of clause 1, wherein the embedding neural network and the generative model have been jointly trained on a plurality of training examples, wherein each training example comprises: (i) a training input that characterizes a training protein and a training ligand, and (ii) a target output based on a joint 3D structure of the training protein and the training ligand.

3. The method of clause 2, wherein the joint training of the embedding neural network and the generative model on the plurality of training examples comprises, for each training example:

processing the training input of the training example using the embedding neural network to generate a protein-ligand embedding of the training protein and the training ligand;
processing the protein-ligand embedding of the training protein and the training ligand using the generative model to generate a predicted output characterizing a predicted joint 3D structure of the training protein and the training ligand of the training example; and
backpropagating gradients of an objective function through the generative model and into the embedding neural network, wherein the objective function measures a discrepancy between: (i) the target output specified by the training example, and (ii) the predicted output generated by the embedding neural network and the generative model for the training example.

4. The method of any preceding clause, wherein generating the property score that defines the predicted property of the protein and the ligand using the protein-ligand embedding comprises:
processing the protein-ligand embedding using a property prediction neural network to generate the property score.

5. The method of clause 4, wherein the property prediction neural network and the embedding neural network have been jointly trained on a plurality of training examples, wherein each training example comprises: (i) a training input that characterizes a training protein and a training ligand, and (ii) a target property score that defines a property of the training protein and the training ligand.

6. The method of clause 5, wherein the joint training of the property prediction neural network and the embedding neural network on the plurality of training examples comprises, for each training example:

processing the training input of the training example using the embedding neural network to generate a protein-ligand embedding of the training protein and the training ligand;
processing the protein-ligand embedding of the training protein and the training ligand using the property prediction neural network to generate a predicted property score for the training protein and the training ligand of the training example; and
backpropagating gradients of an objective function through the property prediction neural network and into the embedding neural network, wherein the objective function measures a discrepancy between: (i) the target property score specified by the training example, and (ii) the predicted property score generated by the embedding neural network and the property prediction neural network for the training example.

7. The method of any preceding clause, wherein generating the property score that defines the predicted property of the protein and the ligand using the protein-ligand embedding comprises:

generating, using the generative model and while the generative model is conditioned on the protein-ligand embedding, a predicted joint 3D structure of the protein and the ligand, wherein the predicted joint 3D structure of the protein and the ligand defines a respective predicted three-dimensional spatial location of each atom in the protein and of each atom in the ligand; and generating the property score that defines the predicted property of the protein and the ligand using the predicted joint 3D structure of the protein and the ligand.

8. The method of clause 7, wherein generating the property score that defines the predicted property of the protein and the ligand using the predicted joint 3D structure of the protein and the ligand comprises:

generating data defining a graph representing at least a portion of the predicted joint 3D structure of the protein and the ligand; and processing the graph representing at least the portion of the predicted joint 3D structure of the protein and the ligand using a graph neural network to generate the property score that defines the predicted property of the protein and the ligand.

9. The method of clause 8, wherein the graph neural network comprises a plurality of message passing layers.

10. The method of any one of clauses 8-9, wherein the graph representing at least the portion of the predicted joint 3D structure of the protein and the ligand comprises: (i) a sets of nodes, and (ii) a set of edges, wherein:

the set of nodes comprises a plurality of atom nodes that each represent a respective atom in the protein or in the ligand; and each edge connects a respective pair of nodes.

11. The method of clause 10, wherein generating the data defining the graph representing at least the portion of the predicted joint 3D structure of the protein and the ligand comprises: generating the set of edges based at least in part on 3D spatial distances between pairs of atoms in the protein and in the ligand.

12. The method of clause 11, wherein generating the set of edges based at least in part on 3D spatial distances between pairs of atoms in the protein and in the ligand comprises, for each pair of atoms that comprises a respective first atom in the protein or in the ligand and a respective second atom in the protein or in the ligand: determining that an atom node representing the first atom and an atom node representing the second atom should be connected by an edge if a 3D spatial distance between the first atom and the second atom satisfies a threshold.

13. The method of any one of clauses 10-12, wherein the set of nodes in the graph further comprises a plurality of super nodes, wherein the plurality of super nodes comprises a respective super node representing each of a plurality of amino acid residues in the protein; and wherein the set of edges in the graph comprises, for each atom node in the graph that represents an atom in the protein, a respective edge between the atom node and a corresponding super node representing an amino acid residue that includes the atom.

14. The method of clause 13, wherein the ligand comprises a plurality of structural motifs, and wherein the plurality of super nodes further comprises a respective super node representing each structural motif in the ligand; and wherein the set of edges in the graph comprises, for each atom node in the graph that represents an atom in the ligand, a respective edge between the atom node and a corresponding super node representing a structural motif that includes the atom.

15. The method of clause 14, wherein the set of edges comprises a respective edge between each pair of super nodes from the plurality of super nodes included in the graph.

16. The method of any preceding clause, wherein the generative model is a generative diffusion model that comprises a denoising neural network.

17. The method of clause 16, wherein generating the property score that defines the predicted property of the protein and the ligand using the protein-ligand embedding comprises:

generating positional data defining a respective initial position of each atom in a complex comprising the protein and the ligand;
generating property data defining a respective initial predicted property of the protein and the ligand;
denoising the positional data and the property data over a sequence of time steps using the denoising neural network and while the denoising neural network is conditioned on the protein - ligand embedding;
wherein, after a final time step in the sequence of time steps:

the positional data defines the predicted joint 3D structure of the protein and the ligand; and
the property data defines the property score.

18. The method of clause 17, wherein denoising the positional data and the property data over the sequence of time steps using the denoising neural network and while the denoising neural network is conditioned on the protein - ligand embedding comprises, at each of one or more time steps in the sequence of time steps:

receiving current positional data that defines a respective current position of each atom in the complex at the time step;
receiving current property data that defines a current predicted property of the protein and the ligand;
generating a denoising output using the denoising neural network and while the denoising neural network is conditioned on the protein - ligand embedding; and
generating positional data that defines a respective position of each atom in the complex at a next time step using the denoising output; and
generating property data that defines a predicted property of the protein and the ligand at the next time step using the denoising output.

19. The method of clause 18, wherein the denoising output comprises: (i) a respective predicted error in the current position of each atom in the complex at the time step, and (ii) a respective predicted error in the current predicted property score of the protein and the ligand at the time step.

20. The method of any one of clauses 18-19, wherein generating the denoising output using the denoising neural network and while the denoising neural network is conditioned on the protein - ligand embedding comprises:

generating a set of embeddings using an encoder block of the denoising neural network, wherein the set of embeddings comprises:

a plurality of atom embeddings, wherein each atom embedding represents one or more respective atoms in the complex and is based at least in part on the respective current spatial position of the one or more atoms at the time step; and
a property embedding that is based at least in part on the current predicted property of the protein and the ligand at the time step;

processing the set of embeddings using an update block of the denoising neural network to generate a set of updated embeddings; and
processing the set of updated embeddings to generate the denoising output.

21. The method of clause 20, wherein the update block of the denoising neural network comprises a sequence of self-attention blocks;

wherein each of the self-attention blocks are configured to

receive a set of current embeddings that comprises a plurality of current atom embeddings and a current property score embedding; and
apply one or more self-attention operations to the set of current embeddings to update the set of current embeddings;

wherein each of the one or more self-attention operations are conditioned on the protein - ligand embedding.

22. The method of any preceding clause, wherein the network input comprises data defining each of one or more amino acid sequences of the protein.

23. The method of any preceding clause, wherein the network input comprises a text string defining a chemical structure of the ligand.

24. The method of any preceding clause, wherein the ligand is a small molecule.

25. The method of any preceding clause, wherein the protein comprises an enzyme, receptor, or signaling protein that has been identified as being involved in a disease process.

26. The method of any preceding clause, wherein the property score is a binding affinity score that defines a predicted binding affinity of the protein and the ligand, and wherein generating the property score that defines the predicted property of the protein and the ligand using the protein-ligand embedding comprises:

   conditioning the generation of the property score on data specifying a type of binding affinity assay; and
   wherein the property score defines the predicted binding affinity of the protein and the ligand as measured by the specified type of binding affinity assay.

27. The method of clause 26, wherein type of binding affinity assay is: a surface plasmon resonance (SPR) assay, or an isothermal titration calorimetry (ITC) assay, or a fluorescence polarization (FP) assay, or an enzyme-linked immunosorbent assay (ELISA), or a radioligand binding assay, or a bioluminescence resonance energy transfer (BERT) assay.

28. The method of any preceding clause, wherein the property score defines one or more of: a likelihood of a binding event that involves the protein and the ligand; or a binding affinity of the protein and the ligand; or a likelihood that the ligand is an agonist for the protein; or a likelihood that the ligand is an antagonist for the protein; or a predicted potency of the ligand when acting on the protein; or a predicted inhibitory effect of the ligand when acting on the protein.

29. A method performed by one or more computers, the method comprising:

   obtaining data defining: (i) a protein, and (ii) a set of candidate ligands;
   generating, using the method of any one of clauses 1-28 and for each ligand of the set of ligands, a respective binding affinity score for the ligand that defines a predicted binding affinity of the protein and the ligand; and
   determining a ranking of the set of candidate ligands based on the binding affinity scores for the ligands.

30. The method of clause 29, further comprising:

   selecting one or more candidate ligands from the set of candidate ligands based on the ranking; and
   physically synthesizing the selected candidate ligands.

31. The method of clause 30, further comprising, for each of the selected candidate ligands, performing experiments using physically synthesized instances (i.e., molecules) of the candidate ligand to determine one or more of: an absorption of the candidate ligand, a distribution of the candidate ligand, a metabolism of the candidate ligand, or an excretion of the candidate ligand.

32. The method of any one of clauses 29-31, wherein the ranking of the candidate ligands ranks the candidate ligands from highest predicted binding affinity for the protein to lowest predicted binding affinity for the protein.

33. A method performed by one or more computers, the method comprising:

   obtaining data defining: (i) a set of candidate proteins, and (ii) a ligand;
   generating, using the method of any one of clauses 1-28 and for each candidate protein of the set of candidate proteins, a respective binding affinity score for the candidate protein that defines a predicted binding affinity of the candidate protein and the ligand; and
   determining a ranking of the set of candidate proteins based on the binding affinity scores.

34. The method of clause 33, further comprising:

selecting one or more candidate proteins from the set of candidate proteins based on the ranking; and physically synthesizing the selected candidate proteins.

35. The method of any one of clauses 33-34, wherein the ranking of the candidate proteins ranks the candidate proteins from highest predicted binding affinity for the ligand to lowest predicted binding affinity for the ligand.

36. A system comprising:

one or more computers; and
one or more storage devices communicatively coupled to the one or more computers, wherein the one or more storage devices store instructions that, when executed by the one or more computers, cause the one or more computers to perform operations of the respective method of any one of clauses 1-29 or 33.

37. One or more non-transitory computer storage media storing instructions that when executed by one or more computers cause the one or more computers to perform operations of the respective method of any one of clauses 1-29 or 33.

**Claims**

1. A method performed by one or more computers, the method comprising:

obtaining a network input that characterizes a protein and a ligand;
processing the network input characterizing the protein and the ligand using an embedding neural network to generate a protein-ligand embedding representing the protein and the ligand,
wherein the embedding neural network has been jointly trained with a generative model that is configured to:

receive an input protein-ligand embedding; and
generate, while conditioned on the input protein-ligand embedding, a predicted joint three-dimensional, 3D, structure of an input protein and an input ligand represented by the input protein-ligand embedding; and

generating a property score that defines a predicted property of the protein and the ligand using the protein-ligand embedding.

2. The method of claim 1, wherein the embedding neural network and the generative model have been jointly trained on a plurality of training examples, wherein each training example comprises: (i) a training input that characterizes a training protein and a training ligand, and (ii) a target output based on a joint 3D structure of the training protein and the training ligand and optionally, wherein the joint training of the embedding neural network and the generative model on the plurality of training examples comprises, for each training example:

processing the training input of the training example using the embedding neural network to generate a protein-ligand embedding of the training protein and the training ligand;
processing the protein-ligand embedding of the training protein and the training ligand using the generative model to generate a predicted output characterizing a predicted joint 3D structure of the training protein and the training ligand of the training example; and
backpropagating gradients of an objective function through the generative model and into the embedding neural network, wherein the objective function measures a discrepancy between: (i) the target output specified by the training example, and (ii) the predicted output generated by the embedding neural network and the generative model for the training example.

3. The method of claim 1 or 2, wherein generating the property score that defines the predicted property of the protein and the ligand using the protein-ligand embedding comprises:
processing the protein-ligand embedding using a property prediction neural network to generate the property score; and preferably, wherein the property prediction neural network and the embedding neural network have been jointly trained on a plurality of training examples, wherein each training example comprises: (i) a training input that characterizes a training protein and a training ligand, and (ii) a target property score that defines a property of the training protein and the training ligand; and more preferably wherein, the joint training of the property prediction neural network and the embedding neural network on the plurality of training examples comprises, for each training example:

processing the training input of the training example using the embedding neural network to generate a protein-ligand embedding of the training protein and the training ligand;

processing the protein-ligand embedding of the training protein and the training ligand using the property prediction neural network to generate a predicted property score for the training protein and the training ligand of the training example; and

backpropagating gradients of an objective function through the property prediction neural network and into the embedding neural network, wherein the objective function measures a discrepancy between: (i) the target property score specified by the training example, and (ii) the predicted property score generated by the embedding neural network and the property prediction neural network for the training example.

4. The method of any preceding claim, wherein generating the property score that defines the predicted property of the protein and the ligand using the protein-ligand embedding comprises:

generating, using the generative model and while the generative model is conditioned on the protein-ligand embedding, a predicted joint 3D structure of the protein and the ligand, wherein the predicted joint 3D structure of the protein and the ligand defines a respective predicted three-dimensional spatial location of each atom in the protein and of each atom in the ligand; and generating the property score that defines the predicted property of the protein and the ligand using the predicted joint 3D structure of the protein and the ligand; and

preferably, wherein generating the property score that defines the predicted property of the protein and the ligand using the predicted joint 3D structure of the protein and the ligand comprises:

generating data defining a graph representing at least a portion of the predicted joint 3D structure of the protein and the ligand; and processing the graph representing at least the portion of the predicted joint 3D structure of the protein and the ligand using a graph neural network to generate the property score that defines the predicted property of the protein and the ligand; and

more preferably, wherein the graph neural network comprises a plurality of message passing layers.

5. The method of claim 4, wherein the graph representing at least the portion of the predicted joint 3D structure of the protein and the ligand comprises: (i) a sets of nodes, and (ii) a set of edges, wherein:

the set of nodes comprises a plurality of atom nodes that each represent a respective atom in the protein or in the ligand; and each edge connects a respective pair of nodes and preferably, wherein generating the data defining the graph representing at least the portion of the predicted joint 3D structure of the protein and the ligand comprises:

generating the set of edges based at least in part on 3D spatial distances between pairs of atoms in the protein and in the ligand, and more preferably, wherein generating the set of edges based at least in part on 3D spatial distances between pairs of atoms in the protein and in the ligand comprises, for each pair of atoms that comprises a respective first atom in the protein or in the ligand and a respective second atom in the protein or in the ligand: determining that an atom node representing the first atom and an atom node representing the second atom should be connected by an edge if a 3D spatial distance between the first atom and the second atom satisfies a threshold.

6. The method of claim 5, wherein the set of nodes in the graph further comprises a plurality of super nodes, wherein the plurality of super nodes comprises a respective super node representing each of a plurality of amino acid residues in the protein; and

wherein the set of edges in the graph comprises, for each atom node in the graph that represents an atom in the protein, a respective edge between the atom node and a corresponding super node representing an amino acid residue that includes the atom, and preferably, wherein the ligand comprises a plurality of structural motifs, and wherein the plurality of super nodes further comprises a respective super node representing each structural motif in the ligand; and

wherein the set of edges in the graph comprises, for each atom node in the graph that represents an atom in the ligand, a respective edge between the atom node and a corresponding super node representing a structural motif that includes the atom, and

more preferably, wherein the set of edges comprises a respective edge between each pair of super nodes from the plurality of super nodes included in the graph.

7. The method of any preceding claim, wherein the generative model is a generative diffusion model that comprises a denoising neural network.

8. The method of claim 7, wherein generating the property score that defines the predicted property of the protein and the ligand using the protein-ligand embedding comprises:

generating positional data defining a respective initial position of each atom in a complex comprising the protein and the ligand;
generating property data defining a respective initial predicted property of the protein and the ligand;
denoising the positional data and the property data over a sequence of time steps using the denoising neural network and while the denoising neural network is conditioned on the protein - ligand embedding;
wherein, after a final time step in the sequence of time steps:

the positional data defines the predicted joint 3D structure of the protein and the ligand; and
the property data defines the property score, and

preferably, wherein denoising the positional data and the property data over the sequence of time steps using the denoising neural network and while the denoising neural network is conditioned on the protein - ligand embedding comprises, at each of one or more time steps in the sequence of time steps:

receiving current positional data that defines a respective current position of each atom in the complex at the time step;
receiving current property data that defines a current predicted property of the protein and the ligand;
generating a denoising output using the denoising neural network and while the denoising neural network is conditioned on the protein - ligand embedding; and
generating positional data that defines a respective position of each atom in the complex at a next time step using the denoising output; and
generating property data that defines a predicted property of the protein and the ligand at the next time step using the denoising output, and

more preferably, wherein the denoising output comprises: (i) a respective predicted error in the current position of each atom in the complex at the time step, and (ii) a respective predicted error in the current predicted property score of the protein and the ligand at the time step.

9. The method of claim 8, wherein generating the denoising output using the denoising neural network and while the denoising neural network is conditioned on the protein - ligand embedding comprises:

generating a set of embeddings using an encoder block of the denoising neural network, wherein the set of embeddings comprises:

a plurality of atom embeddings, wherein each atom embedding represents one or more respective atoms in the complex and is based at least in part on the respective current spatial position of the one or more atoms at the time step; and
a property embedding that is based at least in part on the current predicted property of the protein and the ligand at the time step;

processing the set of embeddings using an update block of the denoising neural network to generate a set of updated embeddings; and
processing the set of updated embeddings to generate the denoising output, and optionally, wherein the update block of the denoising neural network comprises a sequence of self-attention blocks;
wherein each of the self-attention blocks are configured to

receive a set of current embeddings that comprises a plurality of current atom embeddings and a current property score embedding; and

apply one or more self-attention operations to the set of current embeddings to update the set of current embeddings;

wherein each of the one or more self-attention operations are conditioned on the protein - ligand embedding.

10. The method of any preceding claim, wherein:

(i) the network input comprises data defining each of one or more amino acid sequences of the protein; and/or
(ii) the network input comprises a text string defining a chemical structure of the ligand; and/or
(iii) the ligand is a small molecule; and/or
(iv) the protein comprises an enzyme, receptor, or signaling protein that has been identified as being involved in a disease process; and/or
(v) wherein the property score is a binding affinity score that defines a predicted binding affinity of the protein and the ligand, and wherein generating the property score that defines the predicted property of the protein and the ligand using the protein-ligand embedding comprises:

conditioning the generation of the property score on data specifying a type of binding affinity assay; and
wherein the property score defines the predicted binding affinity of the protein and the ligand as measured by the specified type of binding affinity assay; and/or

(vi) wherein the property score defines one or more of: a likelihood of a binding event that involves the protein and the ligand; or a binding affinity of the protein and the ligand; or a likelihood that the ligand is an agonist for the protein; or a likelihood that the ligand is an antagonist for the protein; or a predicted potency of the ligand when acting on the protein; or a predicted inhibitory effect of the ligand when acting on the protein.

11. A method performed by one or more computers, the method comprising:

obtaining data defining: (i) a protein, and (ii) a set of candidate ligands;
generating, using the method of any one of claims 1-10 and for each ligand of the set of ligands, a respective binding affinity score for the ligand that defines a predicted binding affinity of the protein and the ligand; and
determining a ranking of the set of candidate ligands based on the binding affinity scores for the ligands.

12. A method performed by one or more computers, the method comprising:

obtaining data defining: (i) a set of candidate proteins, and (ii) a ligand;
generating, using the method of any one of claims 1-10 and for each candidate protein of the set of candidate proteins, a respective binding affinity score for the candidate protein that defines a predicted binding affinity of the candidate protein and the ligand; and
determining a ranking of the set of candidate proteins based on the binding affinity scores; and optionally, wherein the ranking of the candidate proteins ranks the candidate proteins from highest predicted binding affinity for the ligand to lowest predicted binding affinity for the ligand.

13. The method of claim 11 or 12, further comprising:

(i) selecting one or more candidate proteins from the set of candidate proteins based on the ranking, and physically synthesizing the selected candidate proteins; or
(ii) selecting one or more candidate ligands from the set of candidate ligands based on the ranking; and physically synthesizing the selected candidate ligands, and optionally, for each of the selected candidate ligands, performing experiments using physically synthesized instances of the candidate ligand to determine one or more of: an absorption of the candidate ligand, a distribution of the candidate ligand, a metabolism of the candidate ligand, or an excretion of the candidate ligand.

14. A system comprising:

one or more computers; and
one or more storage devices communicatively coupled to the one or more computers, wherein the one or more storage devices store instructions that, when executed by the one or more computers, cause the one or more computers to perform operations of the respective method of any one of claims 1-12.

15. One or more non-transitory computer storage media storing instructions that when executed by one or more computers cause the one or more computers to perform operations of the respective method of any one of claims 1-12.

**PROPERTY PREDICTION SYSTEM 100**

FIG. 1A

# EMBEDDING NEURAL NETWORK <u>112</u>

FIG. 1B

# GENERATING JOINT EMBEDDING OF PROTEIN AND LIGAND

130

| |
| --- |
| RECEIVE PROTEIN EMBEDDING AND LIGAND EMBEDDING | 132 |

↓

| CONCATENATE PROTEIN EMBEDDING AND LIGAND EMBEDDING INTO 1D SEQUENCE OF EMBEDDINGS | 134 |

↓

| TRANSFORM 1D SEQUENCE OF EMBEDDINGS INTO 2D ARRAY OF EMBEDDINGS | 136 |

↓

| PROCESS 2D ARRAY OF EMBEDDINGS USING SELF-ATTENTION LAYERS OF FUSION NEURAL NETWORK | 138 |

↓

| OUTPUT 2D ARRAY OF EMBEDDINGS GENERATED BY FUSION NEURAL NETWORK AS PROTEIN – LIGAND EMBEDDING | 140 |

FIG. 1C

PROTEIN

ONE OR MORE
LIGANDS

**AMINO ACID**
**EMBEDDINGS** <u>142</u>

**ATOM EMBEDDINGS** <u>144</u>

146

OUTER
PRODUCT

2D ARRAY OF
EMBEDDINGS
<u>148</u>

<u>150</u>

AMINO ACID –
AMINO ACID
EMBEDDINGS

<u>152</u>

AMINO ACID –
ATOM
EMBEDDINGS

<u>154</u>

AMINO ACID –
ATOM
EMBEDDINGS

<u>156</u>

ATOM – ATOM
EMBEDDINGS

**FIG. 1D**

**GENERATING A PROPERTY SCORE USING A PROPERTY PREDICTION NEURAL NETWORK**

200

GENERATE A PROTEIN-LIGAND EMBEDDDING USING AN EMBEDDING NEURAL NETWORK ⌐202

PROCESS THE PROTEIN-LIGAND EMBEDDING USING A PROPERTY PREDICTION NEURAL NETWORK TO GENERATE A PROPERTY SCORE ⌐204

FIG. 2

JOINTLY TRAINING A PROPERTY NEURAL NETWORK WITH AN
EMBEDDING NEURAL NETWORK

300

PROCESS A TRAINING INPUT OF A TRAINING EXAMPLE USING AN
EMBEDDING NEURAL NETWORK TO GENERATE A PROTEIN-LIGAND
EMBEDDING 302

PROCESS THE PROTEIN-LIGAND EMBEDDING USING A PROPERTY
PREDICTION NEURAL NETWORK TO GENERATE A PREDICTED
PROPERTY SCORE FOR THE TRAINING EXAMPLE 304

BACKPROPAGATE GRADIENTS OF AN OBJECTIVE FUNCTION THROUGH
THE BINDING PREDICTION NEURAL NETWORK AND INTO THE
EMBEDDING NEURAL NETWORK 306

FIG. 3

## GENERATING A PROPERTY SCORE USING A GRAPH NEURAL NETWORK

400

GENERATE A PROTEIN-LIGAND EMBEDDDING USING AN EMBEDDING NEURAL NETWORK — 402

GENERATE, USING A GENERATIVE MODEL AND WHILE THE GENERATIVE MODEL IS CONDITIONED ON A PROTEIN-LIGAND EMBEDDING, A PREDICTED JOINT 3D STRUCTURE OF A PROTEIN AND A LIGAND — 404

GENERATE DATA DEFINING A GRAPH REPRESENTING AT LEAST A PORTION OF THE PREDICTED JOINT 3D STRUCTURE OF THE PROTEIN AND THE LIGAND — 406

PROCESS THE GRAPH USING A GRAPH NEURAL NETWORK TO GENERATE A PROPERTY SCORE THAT DEFINES THE PREDICTED PROPERTY OF THE PROTEIN AND THE LIGAND — 408

FIG. 4A

**GENERATING A GRAPH REPRESENTING THE 3D STRUCTURE OF A PROTEIN-LIGAND COMPLEX**

410

| RECEIVE DATA DEFINING 3D STRUCTURE OF PROTEIN-LIGAND COMPLEX |
|---|

— 412

| INSTANTIATE A SET OF NODES FOR INCLUSION IN THE GRAPH, INCLUDING ATOM NODES AND, OPTIONALLY, SUPER NODES |
|---|

— 414

| INSTANTIATE A SET OF EDGES FOR INCLUSION IN THE GRAPH |
|---|

— 416

| ASSOCIATE A RESPECTIVE SET OF FEATURES WITH EACH NODE IN THE GRAPH, AND OPTIONALLY, WITH EACH EDGE IN THE GRAPH |
|---|

— 418

FIG. 4B

**PROCESSING A GRAPH REPRESENTING THE 3D STRUCTURE OF A PROTEIN-LIGAND COMPLEX TO GENERATE A PREDICTED PROPERTY SCORE**

420

| |
|---|
| RECEIVE GRAPH REPRESENTING 3D STRUCTURE OF PROTEIN-LIGAND COMPLEX — 422 |

↓

| |
|---|
| GENERATE NODE/EDGE EMBEDDINGS USING ENCODER BLOCK OF GRAPH NEURAL NETWORK — 424 |

↓

| |
|---|
| GENERATE UPDATED NODE/EDGE EMBEDDINGS USING MESSAGE PASSING LAYERS OF GRAPH NEURAL NETWORK — 426 |

↓

| |
|---|
| PROCESS UPDATED NODE/EDGE EMBEDDINGS USING OUTPUT BLOCK OF GRAPH NEURAL NETWORK TO GENERATE PREDICTED PROPERTY SCORE — 428 |

**FIG. 4C**

**TRAINING A GRAPH NEURAL NETWORK**

500

| RECEIVE A SET OF TRAINING EXAMPLES | 502 |

↓

| TRAIN A GRAPH NEURAL NETWORK USING THE TRAINING EXAMPLES TO OPTIMIZE AN OBJECTIVE FUNCTION | 504 |

FIG. 5

600

**OPERATIONS OF A GENERATIVE DIFFUSION MODEL**

GENERATE INITIAL POSITIONS FOR ATOMS IN PROTEIN AND LIGAND — 602

GENERATE INITIAL PROPERTY SCORE FOR PROTEIN AND LIGAND — 604

GENERATE DENOISING OUTPUT AT CURRENT TIME STEP USING DENOISING NEURAL NETWORK CONDITIONED ON PROTEIN-LIGAND EMBEDDING — 606

GENERATE INITIAL ESTIMATES OF ATOM POSITIONS AND PROPERTY SCORE USING DENOISING OUTPUT — 608

610 — CURRENT TIME STEP IS FINAL TIME STEP?

612 — OUTPUT PREDICTED JOINT 3D STRUCTURE OF COMPLEX AND PROPERTY SCORE

614 — GENERATE ATOM POSITIONS AND PROPERTY SCORE FOR NEXT TIME STEP USING DIFFUSION SAMPLER

FIG. 6

**GENERATING DENOISING OUTPUT USING DENOISING NEURAL NETWORK**

700

RECEIVE CURRENT POSITION FOR EACH ATOM IN THE COMPLEX — 702

↓

RECEIVE CURRENT PROPERTY SCORE — 704

↓

GENERATE A SET OF EMBEDDINGS INCLUDING ATOM EMBEDDINGS FOR ATOMS IN THE COMPLEX AND A PROPERTY EMBEDDING, OPTIONALLY CONDITIONING ON PROTEIN-LIGAND EMBEDDING — 706

↓

GENERATE UPDATED SET OF EMBEDDINGS, OPTIONALLY CONDITIONING ATTENTION OPERATIONS ON PROTEIN-LIGAND EMBEDDING — 708

↓

PROCESS UPDATED SET OF EMBEDDINGS TO GENERATE DENOISING OUTPUT — 710

FIG. 7A

# CONDITIONING A SELF-ATTENTION OPERATION ON A PROTEIN – LIGAND EMBEDDING

712

```
RECEIVE: (I) A SET OF CURRENT ATOM
EMBEDDINGS, AND (II) A PROTEIN – LIGAND
EMBEDDING                                          714
```

```
GENERATE A SET OF INTERMEDIATE ATTENTION
SCORES BASED ON THE SET OF CURRENT ATOM
EMBEDDINGS                                         716
```

```
GENERATE A SET OF ATTENTION SCORE BIASES
BASED ON THE PROTEIN – LIGAND EMBEDDING           718
```

```
GENERATE A SET OF FINAL ATTENTION SCORES BY
COMBINING: (I) THE INTERMEDIATE ATTENTION
SCORES, AND (II) THE ATTENTION SCORE BIASES       720
```

```
GENERATE A SET OF UPDATED ATOM EMBEDDINGS
USING: (I) THE SET OF CURRENT ATOM
EMBEDDINGS, AND (II) THE SET OF FINAL
ATTENTION SCORES                                  722
```

## FIG. 7B

# TRAINING THE EMBEDDING NEURAL NETWORK AND THE GENERATIVE MODEL

800

RECEIVE PROTEIN – LIGAND COMPLEX DATA  — 802

GENERATE A SET OF TRAINING EXAMPLES  — 804

JOINTLY TRAIN THE EMBEDDING NEURAL NETWORK AND THE GENERATIVE MODEL ON THE SET OF TRAINING EXAMPLES  — 806

## FIG. 8

**JOINTLY TRAINING AN EMBEDDING NEURAL NETWORK AND GENERATIVE DIFFUSION MODEL ON A TRAINING EXAMPLE**

900

| GENERATE PROTEIN-LIGAND EMBEDDING USING AN EMBEDDING NEURAL NETWORK | 902 |

| SAMPLE TIME STEP FROM A SEQUENCE OF DENOISING TIME STEPS | 904 |

| GENERATE A RESPECTIVE NOISY SPATIAL POSITION FOR EACH ATOM IN THE COMPLEX AND A NOISY BINDING AFFINITY BASED ON THE SAMPLED TIME STEP | 906 |

| GENERATE DENOISING OUTPUT USING DENOISING NEURAL NETWORK CONDITIONED ON PROTEIN-LIGAND EMBEDDING | 908 |

| UPDATE PARAMETER VALUES OF EMBEDDING NEURAL NETWORK AND DISFFUSION NEURAL NETWORK USING GRADIENTS OF OBJECTIVE FUNCTION THAT DEPENDS ON THE DENOISING OUTPUT | 910 |

FIG. 9

## EUROPEAN SEARCH REPORT

**Application Number**

EP 25 17 8122

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 11 256 995 B1 (BUCHER ALWIN [GB] ET AL) 22 February 2022 (2022-02-22) | 1-6, 10-15 | INV.<br>G16B15/20 |
| Y | * whole document, in particular: | 7-9 | G06N3/045 |
| | p.46, col.10, line 50ff | | G16B15/30 |
| | p.47, col.11, line 13ff | | G16B20/00 |
| | p.47, col.12, line 64ff | | G16B40/20 |
| | p.48, col.13, line  5ff; figure 7 | | |
| | p.49, col.15, line 54ff | | |
| | p.49, col.15, line 61ff | | ADD. |
| | p.49, col.16, line 54ff | | G06N3/084 |
| | p.50, col.18, line 43ff | | |
| | p.51, col.19, line  4ff | | |
| | p.51, col.20, line 35ff | | |
| | p.51, col.20, line 45ff | | |
| | p.51, col.20, line 66ff | | |
| | p.52, col.22, line 59ff | | |
| | p.52, col.21, line 11ff | | |
| | p.53, col.23, line  4ff | | |
| | p.54, col.26, line 60ff | | |
| | claim1 1, 17,11 * | | |
| | ----- | | TECHNICAL FIELDS SEARCHED (IPC) |
| Y | BAI QIFENG ET AL: "Geometric deep learning methods and applications in 3D structure-based drug design", DRUG DISCOVERY TODAY, ELSEVIER, AMSTERDAM, NL, vol. 29, no. 7, 16 May 2024 (2024-05-16), XP087557448, ISSN: 1359-6446, DOI: 10.1016/J.DRUDIS.2024.104024 [retrieved on 2024-05-16] | 7,8 | G16B<br>G06N |
| A | * whole document, in particular: p.2, Table 1; p.5, col.1, par.1ff; figure 2 p.8, col.2, par. 2ff; figure 4 * | 1-6,9-15 | |
| | ----- | | |
| | -/-- | | |

The present search report has been drawn up for all claims

| Place of search | Date of completion of the search | Examiner |
|---|---|---|
| Munich | 8 October 2025 | Eberhardt, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

**Application Number**

EP 25 17 8122

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ZHENG YANGKUN ET AL: "De Novo Design of Target-Specific Ligands Using BERT-Pretrained Transformer", 25 December 2023 (2023-12-25), PATTERN RECOGNITION AND COMPUTER VISION; [LECTURE NOTES IN COMPUTER SCIENCE; LECT.NOTES COMPUTER], SPRINGER NATURE SINGAPORE, SINGAPORE, PAGE(S) 311 - 322, XP047679133, ISSN: 0302-9743 ISBN: 978-981-99-8548-7 [retrieved on 2023-12-25] | 9 | |
| A | * whole document in particular: p.4, par. 5 * | 1-8, 10-15 | |
| A | WO 2023/104284 A1 (DEEPLAB IKE [GR]) 15 June 2023 (2023-06-15) * the whole document * | 1-15 | |
| A | KNUTSON CARTER ET AL: "Decoding the protein-ligand interactions using parallel graph neural networks", SCIENTIFIC REPORTS, vol. 12, no. 1, 10 May 2022 (2022-05-10), XP093271575, US ISSN: 2045-2322, DOI: 10.1038/s41598-022-10418-2 * the whole document * | 1-15 | **TECHNICAL FIELDS SEARCHED (IPC)** |
| A | CN 114 783 514 A (SHANGHAI TIANWU TECH CO LTD) 22 July 2022 (2022-07-22) * the whole document * | 1-15 | |

The present search report has been drawn up for all claims

| Place of search | Date of completion of the search | Examiner |
|---|---|---|
| Munich | 8 October 2025 | Eberhardt, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 25 17 8122

This annex lists the patent family members relating to the patent documents cited in the above-mentioned European search report.
The members are as contained in the European Patent Office EDP file on
The European Patent Office is in no way liable for these particulars which are merely given for the purpose of information.

08-10-2025

| Patent document cited in search report | | Publication date | Patent family member(s) | Publication date |
|---|---|---|---|---|
| US 11256995 | B1 | 22-02-2022 | NONE | |
| WO 2023104284 | A1 | 15-06-2023 | NONE | |
| CN 114783514 | A | 22-07-2022 | NONE | |

EPO FORM P0459

For more details about this annex : see Official Journal of the European Patent Office, No. 12/82

**EP 4 654 205 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JUMPER et al.** Highly accurate protein structure prediction with AlphaFold. *Nature*, 26 August 2021, vol. 596
- **TIM SALIMANS** ; **JONATHAN HO**. Progressive distillation for fast sampling of diffusion models. *ICLR 2022, arXiv:2202.00512v2*
- **TIM SALIMANS** ; **JONATHAN HO**. Progressive distillation for fast sampling of diffusion models. *ICLR 2022, arXiv:2202.00512v2.*
- **JIAMING SONG** ; **CHENLIN MENG** ; **STEFANO ERMON**. Denoising diffusion implicit models. *ICLR 2021, arXiv:2010.02502v4.*

**Erroneously filed documents**

**Erroneously filed drawings (or parts thereof)**

**BINDING AFFINITY PREDICTION SYSTEM 100**

LIGAND DATA 104

PROTEIN DATA 102

EMBEDDING NEURAL NETWORK 112

PROTEIN-LIGAND EMBEDDING 106

GENERATIVE MODEL 108

PREDICTED JOINT 3D STUCTURE 110

BINDING AFFINITY SCORE PREDICTION MODULE 114

BINDING AFFINITY SCORE 116

FIG. 1

## EMBEDDING NEURAL NETWORK <u>200</u>

FIG. 2

**GENERATING JOINT EMBEDDING OF PROTEIN AND LIGAND(S)**

300 ⟋

| RECEIVE PROTEIN EMBEDDING AND LIGAND EMBEDDING | ⟋302 |

| CONCATENATE PROTEIN EMBEDDING AND LIGAND EMBEDDING INTO 1D SEQUENCE OF EMBEDDINGS | ⟋304 |

| TRANSFORM 1D SEQUENCE OF EMBEDDINGS INTO 2D ARRAY OF EMBEDDINGS | ⟋306 |

| PROCESS 2D ARRAY OF EMBEDDINGS USING SELF-ATTENTION LAYERS OF FUSION NEURAL NETWORK | ⟋308 |

| OUTPUT 2D ARRAY OF EMBEDDINGS GENERATED BY FUSION NEURAL NETWORK AS PROTEIN-LIGAND EMBEDDING | ⟋310 |

**FIG. 3A**

FIG. 3B

# GENERATING BINDING AFFINITY USING A BINDING PREDICTION NEURAL NETWORK

400

| GENERATE A PROTEIN-LIGAND EMBEDDDING USING AN EMBEDDING NEURAL NETWORK | 402 |

| PROCESS THE PROTEIN-LIGAND EMBEDDING USING A BINDING PREDICTION NEURAL NETWORK TO GENERATE A BINDING AFFINITY SCORE | 404 |

FIG. 4

**JOINTLY TRAINING A BINDING PREDICTION NEURAL NETWORK WITH AN EMBEDDING NEURAL NETWORK**

500

| PROCESS A TRAINING INPUT OF A TRAINING EXAMPLE USING AN EMBEDDING NEURAL NETWORK TO GENERATE A PROTEIN-LIGAND EMBEDDING | 502 |

| PROCESS THE PROTEIN-LIGAND EMBEDDING USING A BINDING PREDICTION NEURAL NETWORK TO GENERATE A PREDICTED BINDING AFFINITY SCORE FOR THE TRAINING EXAMPLE | 504 |

| BACKPROPAGATE GRADIENTS OF AN OBJECTIVE FUNCTION THROUGH THE BINDING PREDICTION NEURAL NETWORK AND INTO THE EMBEDDING NEURAL NETWORK | 506 |

FIG. 5

GENERATING BINDING AFFINITY USING A GRAPH NEURAL NETWORK

600

| GENERATE A PROTEIN-LIGAND EMBEDDDING USING AN EMBEDDING NEURAL NETWORK | 602 |

| GENERATE, USING A GENERATIVE MODEL AND WHILE THE GENERATIVE MODEL IS CONDITIONED ON A PROTEIN-LIGAND EMBEDDING, A PREDICTED JOINT 3D STRUCTURE OF A PROTEIN AND A LIGAND | 604 |

| GENERATE DATA DEFINING A GRAPH REPRESENTING AT LEAST A PORTION OF THE PREDICTED JOINT 3D STRUCTURE OF THE PROTEIN AND THE LIGAND | 606 |

| PROCESS THE GRAPH USING A GRAPH NEURAL NETWORK TO GENERATE A BINDING AFFINITY SCORE THAT DEFINES THE PREDICTED BINDING AFFINITY OF THE PROTEIN AND THE LIGAND | 608 |

FIG. 6

TRAINING A GRAPH NEURAL NETWORK

700

RECEIVE A SET OF TRAINING EXAMPLES — 702

TRAIN A GRAPH NEURAL NETWORK USING THE TRAINING EXAMPLES TO REDUCE A DISCEPANCY — 704

FIG. 7

**GENERATING A GRAPH REPRESENTING AT LEAST A PORTION OF THE PREDICTED JOINT 3D STRUCTURE OF A PROTEIN AND A LIGAND**

RECEIVE JOINT 3D STRUCTURE OF PROTEIN AND LIGAND FROM GENERATIVE MODEL — 802

INSTANTIATE ATOM NODES IN THE GRAPH REPRESENTING ATOMS IN THE PROTEIN AND IN THE LIGAND — 804

INSTANTIATE EDGES — 806

ASSOCIATE A SET OF FEATURES WITH EACH NODE IN THE GRAPH — 808

FIG. 8

**PROCESSING A GRAPH USING A GRAPH NEURAL NETWORK**

900

PROCESSES A FEATURE SET ASSOCIATED WITH EACH NODE IN A GRAPH TO GENERATE A NODE EMBEDDING FOR THE NODE USING AN ENCODER BLOCK — 902

UPDATE EACH NODE EMBEDDING USING MESSAGE PASSING OPERATIONS THAT ARE CONDITIONED ON THE TOPOLOGY OF THE GRAPH USING A SEQUENCE OF MESSAGE PASSING LAYERS — 904

PROCESS THE NODE EMBEDDINGS PRODUCED BY THE FINAL MESSAGE PASSING LAYER TO GENERATE THE PREDICTED BINDING AFFINITY USING AN OUTPUT BLOCK — 906

FIG. 9

1000

**OPERATIONS OF A GENERATIVE DIFFUSION MODEL**

GENERATE INITIAL POSITIONS FOR ATOMS IN PROTEIN AND LIGAND — 1002

GENERATE INITIAL BINDING AFFINITIES FOR PROTEIN AND LIGAND — 1004

GENERATE DENOISING OUTPUT AT CURRENT TIME STEP USING DENOISING NEURAL NETWORK CONDITIONED ON PROTEIN-LIGAND EMBEDDING — 1006

GENERATE INITIAL ESTIMATES OF ATOM POSITIONS AND BINDING AFFINITY USING DENOISING OUTPUT — 1008

1110 — CURRENT TIME STEP IS FINAL TIME STEP?

1114 — GENERATE ATOM POSITIONS AND BINDING AFFINITY FOR NEXT TIME STEP USING DIFFUSION SAMPLER

1112 — OUTPUT PREDICTED JOINT 3D STRUCTURE OF COMPLEX AND BINDING AFFINITY SCORE

FIG. 10

## GENERATING DENOISING OUTPUT USING DENOISING NEURAL NETWORK

1100

| RECEIVE CURRENT POSITION FOR EACH ATOM IN THE COMPLEX | 1102 |

| RECEIVE CURRENT BINDING AFFINITY OF A PROTEIN AND LIGAND | 1104 |

| GENERATE A SET OF EMBEDDINGS INCLUDING ATOM EMBEDDINGS FOR ATOMS IN THE COMPLEX AND A BINDING AFFINITY EMBEDDING, OPTIONALLY CONDITIONING ON PROTEIN-LIGAND EMBEDDING | 1106 |

| GENERATE UPDATED SET OF EMBEDDINGS, OPTIONALLY CONDITIONING ATTENTION OPERATIONS ON PROTEIN-LIGAND EMBEDDING | 1108 |

| PROCESS UPDATED SET OF EMBEDDINGS TO GENERATE DENOISING OUTPUT | 1110 |

FIG. 11

## CONDITIONING A SELF-ATTTENTION OPERATION ON A
## PROTEIN-LIGAND EMBEDDING

1200

RECEIVE: (I) A SET OF CURRENT ATOM EMBEDDINGS, AND (II) A PROTEIN-LIGAND EMBEDDING — 1202

GENERATE A SET OF INTERMEDIATE ATTENTION SCORES BASED ON THE SET OF CURRWNT ATOM EMBEDDINGS — 1204

GENERATE A SET OF ATTENTION SCORE BIASES BASED ON THE PROTEIN-LIGAND EMBEDDING — 1206

GENERATE A SET OF FINAL ATTENTION SCORES BY COMBINING: (I) THE INTERMEDIATE ATTENTION SCORES, AND (II) THE ATTENTION SCORE BIASES — 1208

GENERATE A SET OF UPDATED ATOM EMBEDDINGS USING: (I) THE SET OF CURRENT ATOM EMBEDDINGS, AND (II) THE SET OF FINAL ATTENTION SCORES — 1210

FIG. 12

## TRAINING THE EMBEDDING NEURAL NETWORK AND THE GENERATIVE MODEL

1300

RECEIVE PROTEIN-LIGAND COMPLEX DATA — 1302

GENERATE A SET OF TRAINING EXAMPLES — 1304

JOINTLY TRAIN THE EMBEDDING NEURAL NETWORK AND THE GENERATIVE MODEL ON THE SET OF TRAINING EXAMPLES — 1306

FIG. 13

## JOINTLY TRAINING AN EMBEDDING NEURAL NETWORK AND GENERATIVE DIFFUSION MODEL ON A TRAINING EXAMPLE

1400

| GENERATE PROTEIN-LIGAND EMBEDDING USING AN EMBEDDING NEURAL NETWORK | — 1402 |

↓

| SAMPLE TIME STEP FROM A SEQUENCE OF DENOISING TIME STEPS | — 1404 |

↓

| GENERATE A RESPECTIVE NOISY SPATIAL POSITION FOR EACH ATOM IN THE COMPLEX AND A NOISY BINDING AFFINITY BASED ON THE SAMPLED TIME STEP | — 1406 |

↓

| GENERATE DENOISING OUTPUT USING DENOISING NEURAL NETWORK CONDITIONED ON PROTEIN-LIGAND EMBEDDING | — 1408 |

↓

| UPDATE PARAMETER VALUES OF EMBEDDING NEURAL NETWORK AND DISFFUSION NEURAL NETWORK USING GRADIENTS OF OBJECTIVE FUNCTION THAT DEPENDS ON THE DENOISING OUTPUT | — 1410 |

FIG. 14

FIG. 15